(19) Europäisches Patentamt / European Patent Office / Office européen des brevets

(11) **EP 2 953 967 B1**

(12) **EUROPEAN PATENT SPECIFICATION**

(45) Date of publication and mention
of the grant of the patent:
**01.05.2019 Bulletin 2019/18**

(21) Application number: **14749330.8**

(22) Date of filing: **07.02.2014**

(51) Int Cl.:
***C07K 14/11*** *(2006.01)*

(86) International application number:
**PCT/US2014/015397**

(87) International publication number:
**WO 2014/124319 (14.08.2014 Gazette 2014/33)**

(54) **HUMAN ADAPTATION OF H5 INFLUENZA**

MENSCHLICHE ANPASSUNG VON H5-INFLUENZA

ADAPTATION DU VIRUS DE LA GRIPPE H5 À L'HOMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **07.02.2013 US 201361762103 P**

(43) Date of publication of application:
**16.12.2015 Bulletin 2015/51**

(73) Proprietor: **Massachusetts Institute of Technology
Cambridge, MA 02139 (US)**

(72) Inventors:
• **SASISEKHARAN, Ram
Bedford
Massachusetts 01730 (US)**
• **RAMAN, Rahul
Waltham
Massachusetts 02452 (US)**
• **THARAKARAMAN, Kannan
Arlington
Massachusetts 02474 (US)**
• **VISWANATHAN, Karthik
Waltham
Massachusetts 02451 (US)**
• **STEBBINS, Nathan Wilson
Cambridge
Massachusetts 02139 (US)**

(74) Representative: **Cornish, Kristina Victoria Joy et al
Kilburn & Strode LLP
Lacon London
84 Theobalds Road
London WC1X 8NL (GB)**

(56) References cited:
**WO-A2-2012/047941**

• **MASAKI IMAI ET AL: "Experimental adaptation of an influenza H5 HA confers respiratory droplet transmission to a reassortant H5 HA/H1N1 virus in ferrets", NATURE, 2 May 2012 (2012-05-02), XP055282774, United Kingdom ISSN: 0028-0836, DOI: 10.1038/nature10831**
• **S. HERFST ET AL: "Airborne Transmission of Influenza A/H5N1 Virus Between Ferrets", SCIENCE, vol. 336, no. 6088, 22 June 2012 (2012-06-22), pages 1534-1541, XP055282780, US ISSN: 0036-8075, DOI: 10.1126/science.1213362**
• **STEVENS J ET AL: "Recent Avian H5N1 Viruses Exhibit Increased Propensity for Acquiring Human Receptor Specificity", JOURNAL OF MOLECULAR BIOLOGY, ACADEMIC PRESS, UNITED KINGDOM, vol. 381, no. 5, 19 September 2008 (2008-09-19), pages 1382-1394, XP023783534, ISSN: 0022-2836, DOI: 10.1016/J.JMB.2008.04.016 [retrieved on 2008-04-11]**
• **R. A. M. FOUCHIER ET AL: "Restricted Data on Influenza H5N1 Virus Transmission", SCIENCE, vol. 335, no. 6069, 10 February 2012 (2012-02-10), pages 662-663, XP55283175, US ISSN: 0036-8075, DOI: 10.1126/science.1218376**

(Cont. next page)

- C. A. RUSSELL ET AL: "The Potential for Respiratory Droplet-Transmissible A/H5N1 Influenza Virus to Evolve in a Mammalian Host", SCIENCE, vol. 336, no. 6088, 22 June 2012 (2012-06-22), pages 1541-1547, XP055282862, US ISSN: 0036-8075, DOI: 10.1126/science.1222526
- THARAKARAMAN KANNAN ET AL: "Structural Determinants for Naturally Evolving H5N1 Hemagglutinin to Switch Its Receptor Specificity", CELL, CELL PRESS, US, vol. 153, no. 7, 6 June 2013 (2013-06-06), pages 1475-1485, XP028572606, ISSN: 0092-8674, DOI: 10.1016/J.CELL.2013.05.035
- DATABASE UniProt [Online] 15 March 2005 (2005-03-15), "Hemagglutinin sequence of influenza virus strain A/Viet Nam/1203/2004", XP002759302, retrieved from EBI accession no. UNIPROT:Q5EP31 Database accession no. Q5EP31

**Description**

**Background**

Influenza, commonly referred to as the flu, is an infectious disease caused by RNA viruses that commonly infect birds and mammals. Avian influenza, including the H5N1 strain, is a highly contagious and potentially fatal pathogen, but it currently has only a limited ability to infect humans. However, avian flu viruses are known to accumulate mutations that may alter host specificity and potentially allow human infection. Two of the major flu pandemics of the last century originated from avian flu viruses that changed their genetic makeup to allow for human infection.

Given the constant evolution of influenza viruses, there is a concern that current avian influenza strains might accumulate mutations that alter their host specificity and allow them to infect humans. The costs of an avian flu pandemic are likely to be significant, in 2005; the threat of such a pandemic resulted in billions of dollars being spent by national governments in trying to develop strategies to manage and combat a potential pandemic. Accordingly, improved surveillance techniques and methods of predicting high risk strains of influenza may have value in preventing or minimizing the risk of a human pandemic. There is a well-recognized need for the development of therapeutic agents, specifically including vaccines, for the treatment and/or prevention of influenza infection, particularly of humans. There is also a need for improved surveillance technologies for identifying and/or characterizing emerging strains and thein infectivity characteristics.

**Summary**

The present invention provides compositions and methods for use in detection, treatment, and/or prevention of influenza transmission and/or infection.

In some embodiments, the present invention provides therapeutic agents, such as vaccine compositions, for treating or preventing influenza infection and/or transmission, particularly in humans. For example, the present disclosure describes HA polypeptides, and in particular provides H5 HA polypeptides, whose amino acid sequence shows a high degree of sequence identity with a reference HA (e.g., a reference H5), or relevant portion thereof, but differs in the presence or absence of certain defined sequence features. In general, the reference HA is one that does not mediate significant human infection and/or transmission (e.g., when tested in one or more established or described assay systems for assessing such human infection and/or transmission). In some embodiments, a provided HA polypeptide is one that mediates human infection and/or transmission. In some embodiments, a provided HA polypeptide shows human infection and/or transmission characteristics comparable to those of a reference HA that is known to mediate such human infection and/or transmission.

Thus, the present disclosure provides certain HA polypeptides and relevant fragments thereof, compositions containing them, and methods of making or using them.

The invention also provides agents that detect provided HA polypeptides and fragments, for example by direct binding thereto. In some embodiments, such detecting agents are or comprise antibodies that bind directly to one or more provided HA polypeptides. In some embodiments, detecting agents discriminate between a particular provided HA polypeptide and one or more reference HAs. In some embodiments, detecting agents discriminate between a particular provided HA polypeptide and one or more reference HAs even when the provided HA polypeptide sequence differs from that of the reference HA only in the presence or absence of one or more features as set forth herein; in some such embodiments, the detecting agent discriminates between diagnostic kits, methods of making vaccine compositions. In some embodiments, a binding agent distinguishes between a particular provided HA polypeptide and one or more reference HAs even when the provided HA polypeptide sequence differs from that of the reference HA only in the presence or absence of 1, 2, 3, 4, or 5 such features. In some embodiments, a binding agent distinguishes between a particular provided HA polypeptide and one or more reference HAs even when the provided HA polypeptide sequence differs from that of the reference HA only in the presence or absence of a single feature as described herein.

In some embodiments, the present disclosure provides techniques and reagents for detecting, characterizing, and/or monitoring influenza infection. In some such embodiments, provided techniques and reagents are utilized to detect, characterize, and/or monitor influenza strains present in a single individual organism, (e.g, in a single human being). In some embodiments, provided techniques and reagents are utilized to detect, characterize, and/or monitor influenza strains present in a population of organisms, (e.g, of human beings). In some embodiments, provided techniques and reagents are utilized to detect, characterize, and/or monitor influenza strains present in an area or environment.

**Brief Description of the Drawing**

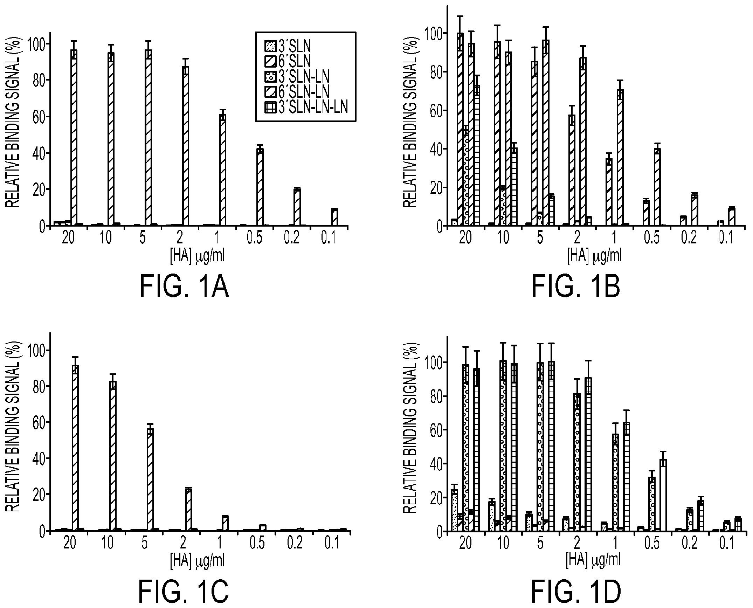

FIG. 1: FIG. 1, Panels 1A-1C illustrates the binding profile of exemplary H1, H2, and H5 pandemic HAs to human and/or avian receptors. **A.** A/South Carolina/1/1918 binding affinity to human receptors (6'SLN-LN) and avian receptors (3'SLN-LN)**B**. A/Albany/6/1958 (Alb58) binding affinity to human receptors (6'SLN-LN) and avian receptors (3'SLN-LN). **C.** A/California/04/2009 binding affinity to human receptors (6'SLN-LN) and avian receptors (3'SLN-LN). Panel **D** illustrates the binding profile of a Viet04 H5 HA strain after introduction of an LS mutation to human receptors (6'SLN-LN) and avian receptors (3'SLN-LN).

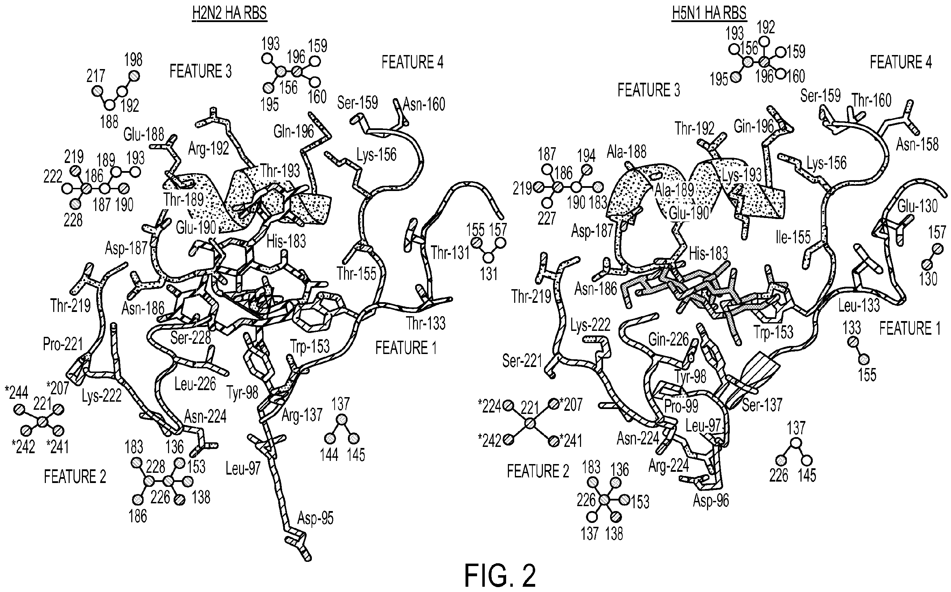

FIG. 2: illustrates a comparison between a representative H2 receptor binding site and a representative H5 receptor binding site.

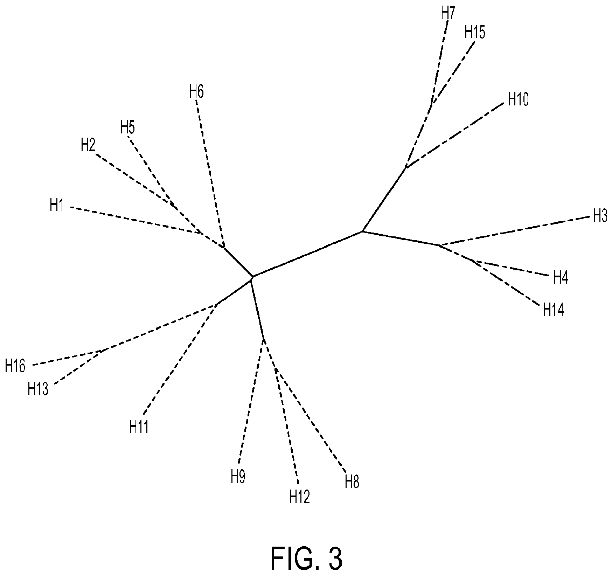

FIG. 3: shows a phylogenetic tree of several influenza subtypes. Closely related subtypes are located on branches close to one another.

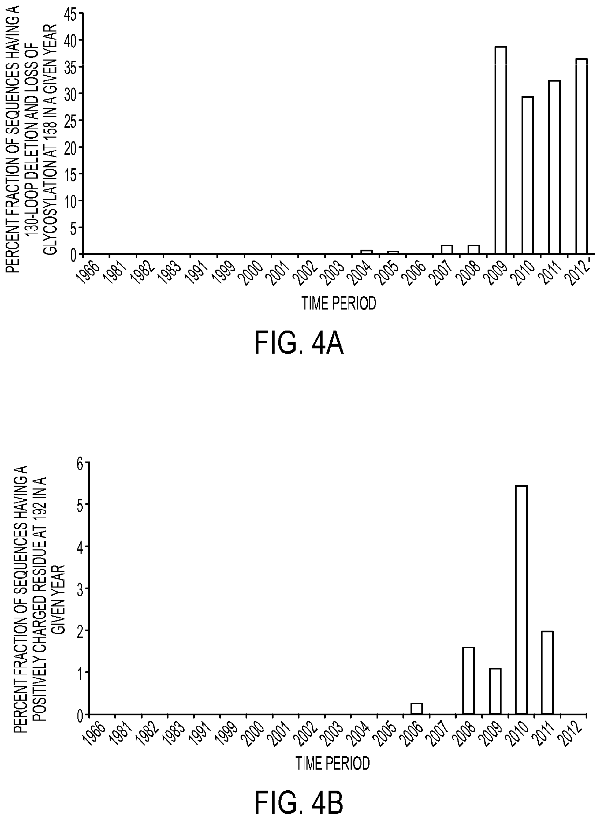

FIG. 4: FIG. 4, Panels 4A and 4B show of the presence or absence of specific RBS features in H5 HA strains over time. **A.** Percent fraction of avian and human H5N1 isolates whose HA has acquired two specific amino acid changes to match features of an H2 HA RBS over time. **B.** Percent fraction of avian and human isolates whose HA has acquired amino acid changes to match a feature of an H2 HA RBS over time.

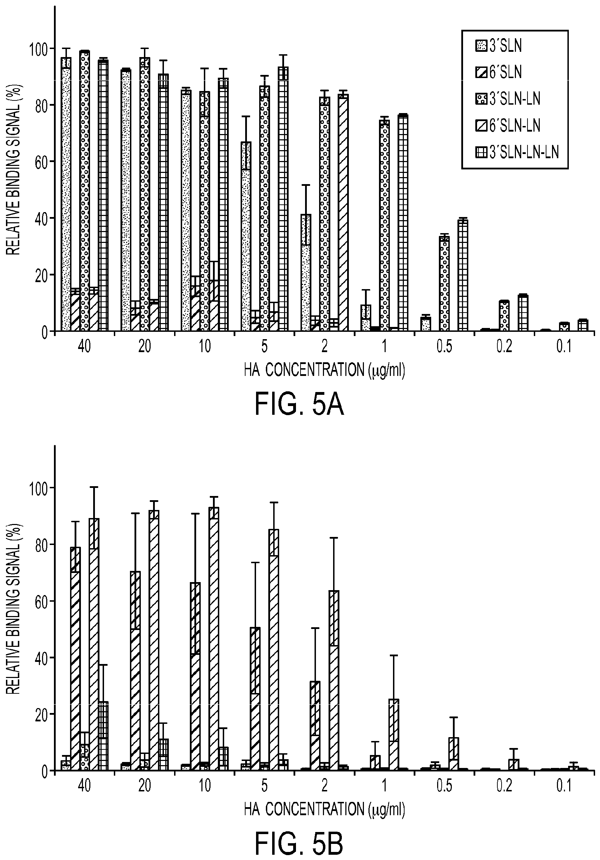

FIG. 5: Panels 5A and 5B demonstrate binding profiles of a wild type influenza virus and a version mutated in accordance with some embodiments. **A.** shows dose-dependent direct binding of wild type Viet04 HA to several receptors. **B.** shows dose-dependent direct binding of V2.3 variant of Viet04 HA to several receptors. Note that the variant form quantitatively switches its binding preference, i.e. shows high affinity binding to human receptors specifically 6'SLN-LN and minimal binding to avian receptors when compared to wild-type Viet04 HA.

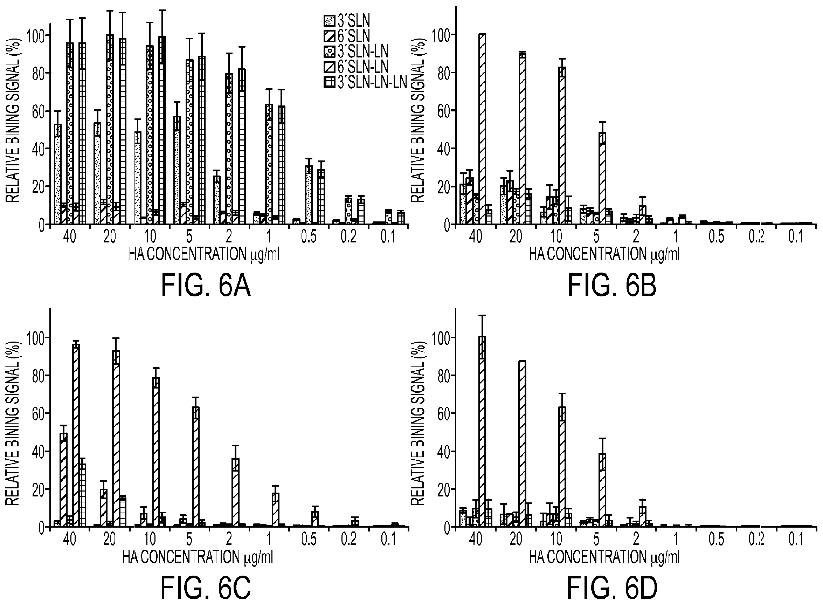

FIG. 6: Panel **A** shows an exemplary dose dependent direct glycan array binding profiles of Egy09 H5 HA, which has naturally evolved to match Features 1 and 4. With the exception of 3'SLN, binding to avian receptors by this HA is similar to that of Viet04. **B,** shows an exemplary dose-dependent direct glycan array binding of E4.2 mutant of Egy09 which required fewer amino acid changes to quantitatively switch its binding to human receptor. **C,** shows an exemplary dose-dependent direct glycan array binding of Egy09 H5 HA with only two amino acid mutations Asn-224→Lys/Gln-226→Leu to match Features 1, 2 and 4. **D,** shows an exemplary dose dependent direct binding of dkEgy10 E5.1 mutant with a single mutation Gln226→Leu to match Features 1,2 and 4.

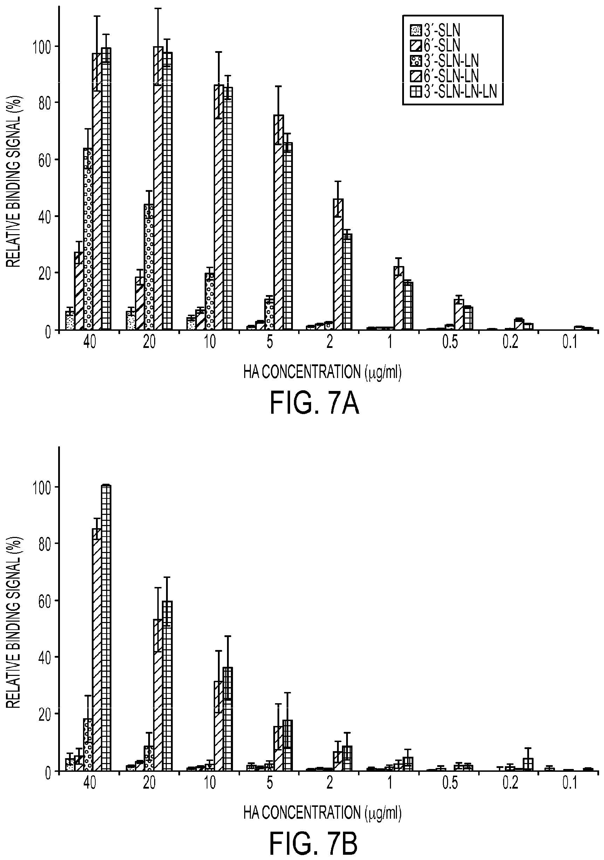

FIG. 7: FIG. 7, Panels 7A and 7B illustrate dose-dependent direct glycan array binding of H5N1 LS mutants belonging to different strains; *x-axis* is HA concentration and *y-axis* is the binding signal value expressed as a percentage of the maximum signal. **A,** A/Egypt/NAMRU-3/06 (Egy06) HA that naturally lacks glycosylation sequon at 158-160 positions. **B,** A/chicken/R2/07, which is a representative recent H5N1 HA belonging to clade 2.2.1 which also naturally lacks the 158-160 glycosylation sequon.

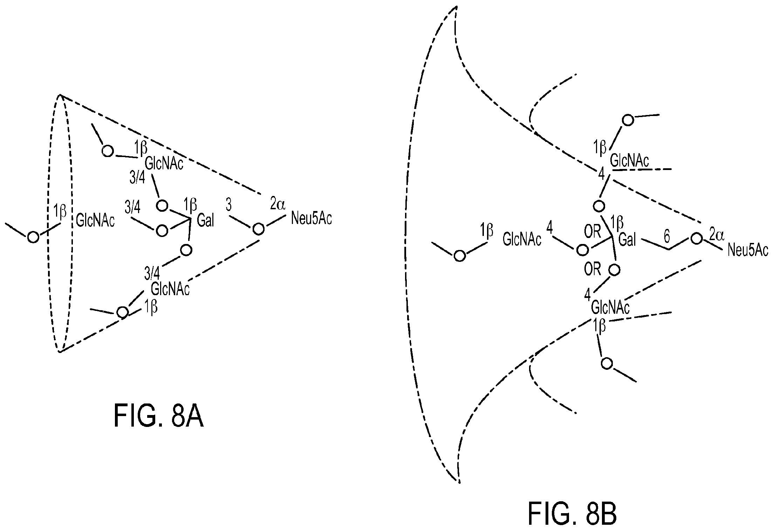

FIG. 8: Framework for understanding glycan receptor specificity. $\alpha$-2-3- and/or $\alpha$-2-6-linked glycans can adopt different topologies. According to the present invention, the ability of an HA polypeptide to bind to certain of these topologies confers upon it the ability to mediate infection of different hosts, for example, humans. As illustrated in Panel A of this figure, the present disclosure defines two particularly relevant topologies, a "cone" topology and an "umbrella" topology. The cone topology can be adopted by $\alpha$-2-3- and/or $\alpha$-2-6-linked glycans, and is typical of short oligosaccharides or branched oligosaccharides attached to a core (although this topology can be adopted by certain long oligosaccharides). The umbrella topology can only be adopted by $\alpha$-2-6-linked glycans (presumably due to the increased conformational plurality afforded by the extra C5-C6 bond that is present in the $\alpha$-2-6 linkage), and is predominantly adopted by long oligosaccharides or branched glycans with long oligosaccharide branches, particularly containing the motif Neu5Ac $\alpha$ 2-6Gal $\beta$ 1-3/4GlcNAc-. As described herein, ability of HA polypeptides to bind the umbrella glycan topology, confers binding to human receptors and/or ability to mediate infection of humans. Panel B of this Figure specifically shows the topology of $\alpha$-2-3 and $\alpha$-2-6 as governed by the glycosidic torsion angles of the trisaccharide motifs--Neu5Ac $\alpha$ 2-3Gal $\beta$ 1-3/4GlcNAc and Neu5Ac $\alpha$ 2-6Gal $\beta$ 1-4GlcNAc respectively. A parameter (.theta.)-angle between C2 atom of Neu5Ac and C1 atoms of the subsequent Gal and GlcNAc sugars in these trisaccharide motifs was defined to characterize the topology.

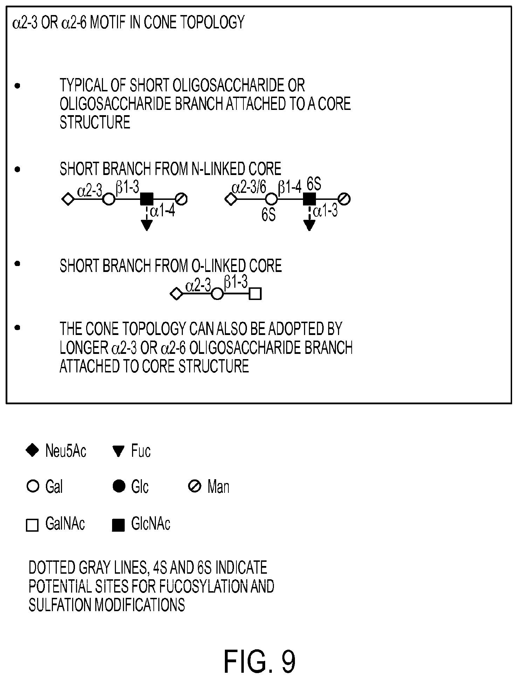

FIG. 9 *Exemplary cone topologies.* This Figure illustrates certain exemplary (but not exhaustive) glycan structures that adopt cone topologies.

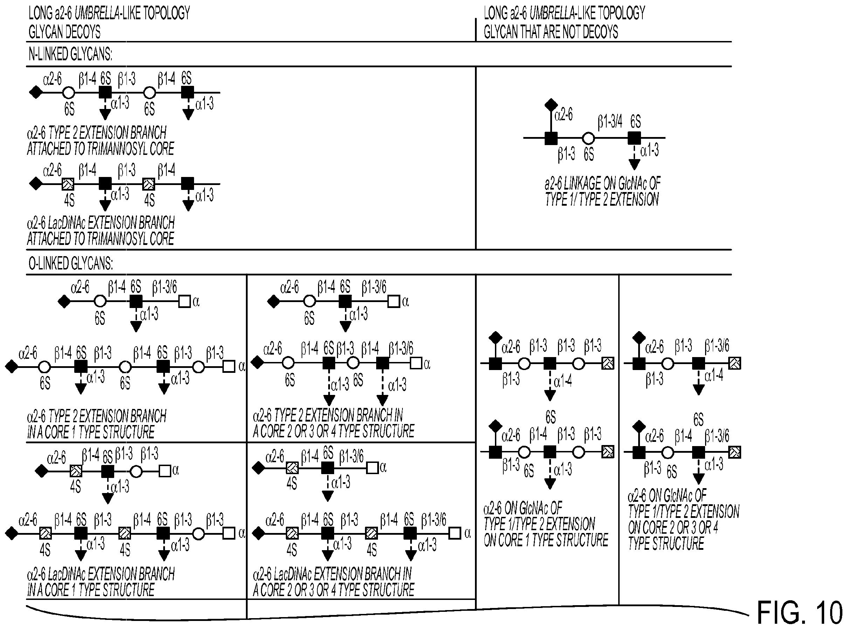

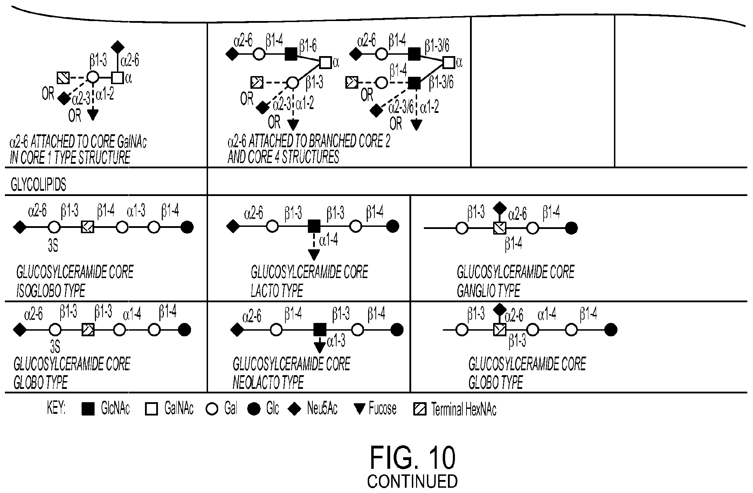

FIG. 10 *Exemplary umbrella topologies.* This Figure shows certain exemplary (but not exhaustive) N- and O-linked glycan structures that can adopt umbrella topologies.

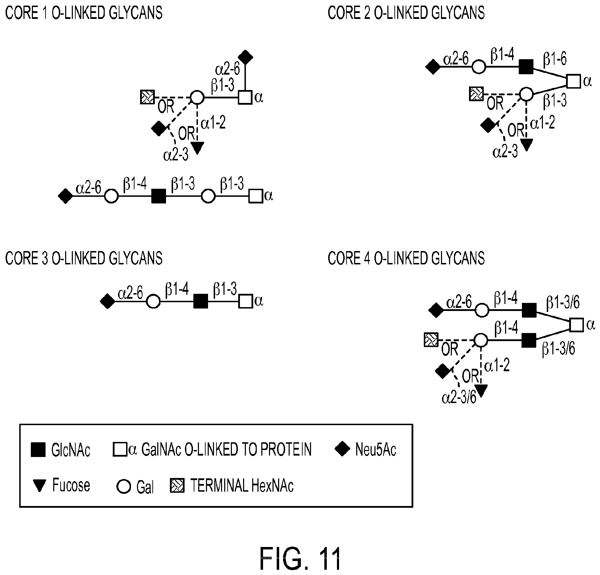

FIG. 11 *Exemplary umbrella topologies.* This Figure shows certain exemplary (but not exhaustive) O-linked glycan structures that can adopt umbrella topologies.

**Description of HA Sequence Elements**

HA Sequence Element 1

*HA Sequence Element 1* is a sequence element corresponding approximately to residues 97-185 (where residue positions are assigned using H3 HA as reference) of many HA proteins found in natural influenza isolates. This sequence element has the basic structure:
C (Y/F) P $X_1$ C $X_2$ W $X_3$ W $X_4$ H H P, wherein:

$X_1$ is approximately 30-45 amino acids long;

$X_2$ is approximately 5-20 amino acids long;

$X_3$ is approximately 25-30 amino acids long; and

$X_4$ is approximately 2 amino acids long.

In some embodiments, $X_1$ is about 35-45, or about 35-43, or about 35, 36, 37, 38, 38, 40, 41, 42, or 43 amino acids long. In some embodiments, $X_2$ is about 9-15, or about 9-14, or about 9, 10, 11, 12, 13, or 14 amino acids long. In some embodiments, $X_3$ is about 26-28, or about 26, 27, or 28 amino acids long. In some embodiments, $X_4$ has the sequence (G/A) (I/V). In some embodiments, $X_4$ has the sequence GI; in some embodiments, $X_4$ has the sequence GV; in some embodiments, $X_4$ has the sequence AI; in some embodiments, $X_4$ has the sequence AV. In some embodiments, HA Sequence Element 1 comprises a disulfide bond. In some embodiments, this disulfide bond bridges residues corresponding to positions 97 and 139 (based on the canonical H3 numbering system utilized herein).

In some embodiments, and particularly in H5 polypeptides, $X_1$ is about 42 amino acids long, and/or $X_2$ is about 13 amino acids long, and/or $X_3$ is about 26 amino acids long.

In some embodiments, and particularly in H5 polypeptides, HA Sequence Element 1 has the structure:
C Y P $X_{1A}$ S SAC $X_2$ W $X_3$ W $X_4$ H H P, wherein:
$X_{1A}$ is approximately 27-42, or approximately 32-42, or approximately 32-40, or approximately 23-38, or approximately 28-38, or approximately 28-36, or approximately 28, 29, 30, 31, 32, 33, 34, 35, 36, 37, 38, 39, or 40 amino acids long, and $X_2$-$X_4$ are as above.

In some embodiments, and particularly in H5 polypeptides, HA Sequence Element 1 has the structure:
C Y P $X_{1A}$ S S A C $X_2$ W L I $X_{3A}$ W $X_4$ H H P, wherein:

$X_{1A}$ is approximately 27-42, or approximately 32-42, or approximately 32-40, or approximately 32, 33, 34, 35, 36, 37, 38, 39, or 40 amino acids long, and

$X_{3A}$ is approximately 23-28, or approximately 24-26, or approximately 24, 25, or 26 amino acids long, and $X_2$ and $X_4$ are as above.

In some embodiments, and particularly in H5 polypeptides, HA Sequence Element 1 is extended (i.e., at a position corresponding to residues 186-193) by the sequence:
NDAAEXX(K/R)

In some embodiments, and particularly in H5 polypeptides, HA Sequence Element 1 includes the sequence:
YEELKHLXSXXNHFEK,
typically within $X_1$, and especially beginning about residue 6 of $X_1$.

HA Sequence Element 2

*HA Sequence Element* 2 is a sequence element corresponding approximately to residues 324-340 (again using a numbering system based on H3 HA) of many HA proteins found in natural influenza isolates. This sequence element has the basic structure:
GAIAGFIE

In some embodiments, HA Sequence Element 2 has the sequence:

P X$_1$G A I A G F I E, wherein:

X$_1$ is approximately 4-14 amino acids long, or about 8-12 amino acids long, or about 12, 11, 10, 9 or 8 amino acids long. In some embodiments, this sequence element provides the HA0 cleavage site, allowing production of HA1 and HA2.

In some embodiments, and particularly in H5 polypeptides, HA Sequence Element 2 has the structure:

P Q R X X X R X X R X$_{1A}$ G A I A G F I E, wherein:

X$_{1A}$ is approximately 3 amino acids long; in some embodiments, X$_{1A}$ is G (L/I) F.

**Definitions**

*Affinity:* As is known in the art, "affinity" is a measure of the tightness with a particular ligand (e.g., an HA polypeptide) binds to its partner (e.g., an HA receptor). Affinities can be measured in different ways. In some embodiments, affinity is measured by a quantitative assay (e.g., glycan binding assays). In some such embodiments, binding partner concentration (e.g., HA receptor, glycan, etc.) may be fixed to be in excess of ligand (e.g., an HA polypeptide) concentration so as to mimic physiological conditions (e.g., viral HA binding to cell surface glycans). Alternatively or additionally, in some embodiments, binding partner (e.g., HA receptor, glycan, etc.) concentration and/or ligand (e.g., an HA polypeptide) concentration may be varied. In some such embodiments, affinity (e.g., binding affinity) may be compared to a reference (e.g., a wild type HA that mediates infection of a humans) under comparable conditions (e.g., concentrations).

*Amino acid residues network:* The term "amino acid residue network" is used to refer to a set of amino acid residues in a polypeptide chain that, although they may be separated from one another along the chain, cluster near one another in space when the chain adopts a folded configuration. Amino acid residues networks on a protein surface are referred to herein as "surface residues networks": those interior to the protein are referred to herein as "core residues networks".

*Antibody:* As used herein, the term "antibody" refers to an immunoglobulin that binds specifically to a particular antigen. The term encompasses immunoglobulins that are naturally produced in that they are generated by an organism reacting to the antigen, and also those that are synthetically produced or engineered. In some embodiments, the term encompasses any polypeptide with immunologloglobulin structural elements sufficient to confer specific binding. An antibody may be monoclonal or polyclonal. An antibody may be a member of any immunoglobulin class, including any of the human classes: IgG, IgM, IgA, and IgD. Suitable antibodies include, but are not limited to, human antibodies, primatized antibodies, chimeric antibodies, bi-specific antibodies, humanized antibodies, conjugated antibodies (*i.e.,* antibodies conjugated or fused to other proteins, radiolabels, cytotoxins), Small Modular ImmunoPharmaceuticals ("SMIPs™"), single chain antibodies, cameloid antibodies, and antibody fragments. As used herein, the term "antibodies" also includes intact monoclonal antibodies, polyclonal antibodies, single domain antibodies (e.g., shark single domain antibodies (e.g., IgNAR or fragments thereof)), multispecific antibodies (*e.g.* bi-specific antibodies) formed from at least two intact antibodies, and antibody fragments so long as they exhibit the desired biological activity.

*Antibody fragment:* As used herein, an "antibody fragment" includes a portion of an intact antibody, such as, for example, the antigen-binding or variable region of an antibody. Examples of antibody fragments include Fab, Fab', F(ab')2, and Fv fragments; triabodies; tetrabodies; linear antibodies; single-chain antibody molecules; and multi specific antibodies formed from antibody fragments. The term "antibody fragment" also includes any synthetic or genetically engineered protein that acts like an antibody by binding to a specific antigen to form a complex. For example, antibody fragments include isolated fragments, "Fv" fragments, consisting of the variable regions of the heavy and light chains, recombinant single chain polypeptide molecules in which light and heavy chain variable regions are connected by a peptide linker ("ScFv proteins"), and minimal recognition units consisting of the amino acid residues that mimic the hypervariable region.

*Antigen:* An "antigen" is a molecule or entity to which an antibody binds. In some embodiments, an antigen is or comprises a polypeptide or portion thereof. In some embodiments, an antigen is a portion of an infectious agent that is recognized by antibodies.

*Approximately:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Associated with:* The term "associated with" is used herein to describe an observed correlation between two items or events. For example, a polypeptide may be considered to be "associated with" a particular infectious agent if its presence or level correlates with a presence or level of the infectious agent. Similarly, a particular collection or pattern of core RBSN scores may be considered to be "associated with" a certain polypeptide structural element (e.g., fold) or functional element if the collection or pattern is observed to correlate with presence of the structural or functional element.

*Binding:* It will be understood that the term "binding", as used herein, typically refers to a non-covalent association between or among two or more entities. "Direct" binding involves physical contact between entities or moieties; indirect binding involves physical interaction by way of physical contact with one or more intermediate entities. Binding between two or more entities can be assessed in any of a variety of contexts - including where interacting entities or moieties are studied in isolation or in the context of more complex systems (e.g., while covalently or otherwise associated with a carrier entity and/or in a biological system or cell.

*Biologically active:* As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Characteristic portion:* As used herein, the term "characteristic portion" is used, in the broadest sense, to refer to a portion of a substance whose presence (or absence) correlates with presence (or absence) of a particular feature, attribute, or activity of the substance. In some embodiments, a characteristic portion of a substance is a portion that is found in the substance and in related substances that share the particular feature, attribute or activity, but not in those that do not share the particular feature, attribute or activity.

*Characteristic pandemic feature:* As used herein the term "characteristic pandemic feature" is one that is found in at least one reference pandemic strain and not in at least one non-pandemic strain. In some embodiments, a characteristic pandemic feature is one that is commonly found in pandemic strains and rarely found in non-pandemic strains. In some embodiments, a characteristic pandemic feature shows prevalence among representative pandemic strains that is at least 30% of that observed among representative non-pandemic strains.

*Characteristic sequence element:* As used herein, the phrase "characteristic sequence element" refers to a sequence element found in a polymer (e.g., in a polypeptide or nucleic acid) that represents a characteristic portion of that polymer. In some embodiments, presence of a characteristic sequence element correlates with presence or level of a particular activity or property of the polymer. In some embodiments, presence (or absence) of a characteristic sequence element defines a particular polymer as a member (or not a member) of a particular family or group of such polymers. A characteristic sequence element typically comprises at least two monomers (e.g., amino acids or nucleotides). In some embodiments, a characteristic sequence element includes at least 2, 3, 4, 5, 6, 7, 8, 9, 10, 11, 12, 13, 14, 15, 20, 25, 30, 35, 40, 45, 50, or more monomers (e.g., contiguously linked monomers). In some embodiments, a characteristic sequence element includes at least first and second stretches of contiguous monomers spaced apart by one or more spacer regions whose length may or may not vary across polymers that share the sequence element.

*Combination therapy:* The term "combination therapy", as used herein, refers to those situations in which two or more different pharmaceutical agents are administered in overlapping regimens so that the subject is simultaneously exposed to both agents.

*Comparable:* The term "comparable", as used herein, refers to two or more agents, entities, situations, sets of conditions, etc that may not be identical to one another but that are sufficiently similar to permit comparison therebetween so that conclusions may reasonably be drawn based on differences or similarities observed. Those of ordinary skill in the art will understand, in context, what degree of identity is required in any given circumstance for two or more such agents, entities, situations, sets of conditions, etc to be considered comparable.

*Corresponding to:* As used herein, the term "corresponding to" is often used to designate the position/identity of an amino acid residue in a polypeptide of interest (e.g., an HA polypeptide). Those of ordinary skill will appreciate that, for purposes of simplicity, residues in a polypeptide are often designated using a canonical numbering system based on a reference related polypeptide, so that an amino acid "corresponding to" a residue at position 190, for example, need not actually be the 190th amino acid in a particular amino acid chain but rather corresponds to the residue found at 190 in the reference polypeptide; those of ordinary skill in the art readily appreciate how to identify "corresponding" amino acids. Typically, residues in HA polypeptides are designated with reference to a canonical wild type H3 HA, and reference in a polypeptide of interest that correspond to resides in the canonical wild type H3 HA are described using the numbering of the residues to which they correspond.

*Degree of separation removed:* As used herein, amino acids that are a "degree of separation removed" are HA amino acids that have indirect effects on glycan binding. For example, one-degree-of-separation-removed amino acids may either: (1) interact with the direct-binding amino acids; and/or (2) otherwise affect the ability of direct-binding amino acids to interact with glycan that is associated with host cell HA receptors; such one-degree-of-separation-removed amino acids may or may not directly bind to glycan themselves. Two-degree-of-separation-removed amino acids either (1) interact with one-degree-of-separation-removed amino acids; and/or (2) otherwise affect the ability of the one-degree-of-separation-removed amino acids to interact with direct-binding amino acids, etc.

*Direct-binding amino acids:* As used herein, the phrase "direct-binding amino acids" refers to HA polypeptide amino acids which interact directly with one or more glycans that is associated with host cell HA receptors.

*Determine:* Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

*Dosage form:* The term "dosage form" is used herein to refer to a physically discrete unit of a therapeutic composition to be administered to a patient. A "unit dosage form" contains an amount of active agent(s) equivalent to a single dose, although it is understood that a prescribing physician may instruct multiple unit dosage forms, or partial unit dosage forms, be administered as a single dose.

*Dosing regimen:* A "dosing regimen" (or "therapeutic regimen"), as that term is used herein, is a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which are separated from one another by a time period of the same length; in some embodiments, a dosing regime comprises a plurality of doses and at least two different time periods separating individual doses. In some embodiments, a dosing regimen is correlated with a particular outcome, event, or probability of such.

*Engineered:* The term "engineered", as used herein, describes a polypeptide whose amino acid sequence has been selected by man and/or whose production required action of the hand of man. For example, an engineered HA polypeptide has an amino acid sequence that differs from the amino acid sequences of HA polypeptides found in natural influenza isolates. In some embodiments, an engineered HA polypeptide has an amino acid sequence that differs from the amino acid sequence of HA polypeptides included in the NCBI database.

*Expression:* The term "expression", when used in reference to a nucleic acid herein, refers to one or more of the following events: (1) production of an RNA transcript of a DNA template (e.g., by transcription); (2) processing of an RNA transcript (e.g., by splicing, editing, 5' cap formation, and/or 3' end formation); (3) translation of an RNA into a polypeptide; and/or (4) post-translational modification of a polypeptide.

*Fold:* The term "fold" is used herein in accordance with its art understood meaning referring to a structural element of a polypeptide that has adopted or can adopt a three-dimensional structure. For example, a fold may be or comprise one or more helices (e.g., alpha-helices) and/or one or more sheets (e.g., beta-sheets).

*Foldome:* As used herein, the term "foldome" refers to the set of polypeptide folds encoded by an organism genome. As will be appreciated by those skilled in the art, in some embodiments, the foldome includes all encoded polypeptide folds; in some embodiments, the foldome includes polypeptide folds present in expressed polypeptides (e.g., in all expressed polypeptides or in polypeptides expressed only under certain conditions such as in certain tissues, at certain times in development, etc.).

*Glycan Array:* As used herein, the term "glycan array" is used to refer to a set of glycans, optionally immobilized on a solid support. In some embodiments, a glycan array is or comprises a collection of glycans present as an organized arrangement or pattern at two or more locations that are physically separated in space. Typically, a glycan array will have at least 4, 8, 16, 24, 48, 96 or several hundred or thousand discrete locations. In general, inventive glycan arrays may have any of a variety of formats. In some embodiments, a glycan array comprises a collection of glycans arranged on a single solid support; in some embodiments, a glycan array comprises a collection of glycans arranged on a plurality of discrete solid supports such as, for example, particulate supports (see, for example, U.S. Patent Application Serial No. 13/087,332). In some embodiments, a glycan array is a microarray in that sample locations are separated from one another by a distance of 50-200 microns or less and/or immobilized glycans are present in the nano to micromolar range or nano to picogram range. Array formats known in the art include, for example, those in which each discrete sample location has a scale of, for example, ten microns. Any of a variety of supports may be utilized in glycan arrays. For example, support materials which may be of use in the present disclosure include hydrophobic membranes, for example, nitrocellulose, PVDF or nylon membranes. Such membranes are well known in the art and can be obtained from, for example, Bio-Rad, Hemel Hempstead, UK. Alternatively or additionally, the support on which glycans are arrayed may comprise a metal oxide. Suitable metal oxides include, but are not limited to, titanium oxide, tantalum oxide, and aluminum oxide. Examples of such materials may be obtained from Sigma-Aldrich Company Ltd, Fancy Road, Poole, Dorset. BH12 4QH UK. Still further, in some embodiments, a support is or comprises a metal oxide gel. A metal oxide gel is considered to provide a large surface area within a given macroscopic area to aid immobilization of the carbohydrate-containing molecules. Additional or alternative support materials which may be used in accordance with the present disclosure include gels, for example silica gels or aluminum oxide gels. Examples of such materials may be obtained from, for example, Merck KGaA, Darmstadt, Germany. In some embodiments, glycan arrays are immobilized on a support that can resist change in size or shape during normal use. For example a support may be a glass slide coated with a component material suitable to be used to array glycans. Also, some composite materials can desirably provide solidity to a support. In some embodiments, glycans are directly attached to the support. In some embodiments, glycans are indirectly attached to the support, for example by being attached to a linker or carrier (e.g., a polypeptide) that is attached to the support. In some embodiments, glycans are covalently attached to the support; in some embodiments, glycans are non-covalently attached to the support. In some embodiments, glycans are reversibly attached to the support (e.g., by way of a cleavable linker and/or a reversible non-covalent interaction). In some embodiments, identity and/or arrangement of glycans in a glycan array is selected so that binding characteristics of polypeptides (e.g., HA polypeptides) of interest can readily be assessed. For example, in some embodiments, glycan arrays for use in accordance with the present disclosure include one or more cone-topology glycans and/or one or more umbrella-topology glycans. In some embodiments, cone topology glycans and umbrella topology glycans are spatially separated from one another. In some embodiments, a plurality of cone topology glycans, or a plurality of umbrella topology glycans, may be spatially localized together (but optionally apart from glycans of the other type). In some embodiments, glycan arrays for use in accordance with the present disclosure include one or more α2-3-linked glycans and/or one or more α 2-6-linked glycans. In some embodiments, α2-3-linked glycans and α2-6-linked glycans are spatially separated from one another. In some embodiments, a plurality of α2-3-linked glycans, or a plurality of α2-6-linked glycans, may be spatially localized together (but optionally apart from glycans of the other type). In some embodiments, such arrays include glycans representative of about 10%, 15%, 20%, 25%, 30% 35%, 40%, 45%, 50%, 55%, 60%, 65%, 70%, 75%, 80%, 85%, 90% 95%, or more of the glycans (e.g., the umbrella glycans, which will often be α2-6 sialylated glycans, particularly long α2-6 sialylated glycans) found on human HA receptors, and particularly on human upper respiratory tract HA receptors. In some embodiments, utilized glycan arrays include some or all of the umbrella and/or cone-topology glycan structures explicitly set forth herein. In some embodiments, arrays include at least about 10%, 15%, 20%, 25%, 30% 35%, 40%, 45%, 50%, 55%, 60%, 65%, 70%, 75%, 80%, 85%, 90% 95%, or more of these glycans.

*Hemagglutinin (HA) polypeptide:* As used herein, the term "hemagglutinin polypeptide" (or "HA polypeptide') refers to a polypeptide whose amino acid sequence includes at least one characteristic sequence of HA. A wide variety of HA sequences from influenza isolates are known in the art; indeed, the National Center for Biotechnology Information (NCBI) maintains a database (http://www.ncbi.nlm.nih.gov/genomes/FLU/) that, as of the filing of the present application included at least 9796 HA sequences. Those of ordinary skill in the art, referring to this database, can readily identify sequences that are characteristic of HA polypeptides generally, and/or of particular HA polypeptides (e.g., H1, H2, H3, H4, H5, H6, H7, H8, H9, H10, H11, H12, H13, H14, H15, or H16 polypeptides; or of HAs that mediate infection of particular hosts, e.g., avian, camel, canine, cat, civet, environment, equine, human, leopard, mink, mouse, seal, stone martin, swine, tiger, whale, etc. For example, in some embodiments, an HA polypeptide includes one or more characteristic sequence elements found between about residues 97 and about 185, about 324 and about 340, about 96 and about 100, and/or about 130 and about 230 of an HA protein found in a natural isolate of an influenza virus. In some embodiments, an HA polypeptide has an amino acid sequence comprising at least one of HA Sequence Elements 1 and 2, as defined herein.

*H5 HA polypeptide:* An "H5 HA polypeptide", as that term is used herein, is an HA polypeptide whose amino acid sequence includes at least one sequence element that is characteristic of H5 and distinguishes H5 from other HA subtypes. Representative such sequence elements can be determined by alignments as will be understood by those skilled in the art.

*High affinity binding:* The term "high affinity binding", as used herein refers to a high degree of tightness with which a particular ligand (e.g., an HA polypeptide) binds to its partner (e.g., an HA receptor). Affinities can be measured by any available method, including those known in the art. In some embodiments, binding is considered to be high affinity if the Kd' is about 500 pM or less (e.g., below about 400 pM, about 300 pM, about 200 pM, about 100 pM, about 90 pM, about 80 pM, about 70 pM, about 60 pM, about 50 pM, about 40 pM, about 30 pM, about 20 pM, about 10 pM, about 5 pM, about 4 pM, about 3 pM, about 2 pM, etc.) in binding assays. In some embodiments, binding is considered to be high affinity if the affinity is stronger (e.g., the Kd' is lower) for a polypeptide of interest than for a selected reference polypeptide. In some embodiments, binding is considered to be high affinity if the ratio of the Kd' for a polypeptide of interest to the Kd' for a selected reference polypeptide is 1:1 or less (e.g., 0.9:1, 0.8:1, 0.7:1, 0.6:1, 0.5:1. 0.4:1, 0.3:1, 0.2:1, 0.1:1, 0.05:1, 0.01:1, or less). In some embodiments, binding is considered to be high affinity if the Kd' for a polypeptide of interest is about 100% or less (e.g., about 99%, about 98%, about 97%, about 96%, about 95%, about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, about 4%, about 3%, about 2%, about 1% or less) of the Kd' for a selected reference polypeptide.

*Host:* The term "host" is used herein to refer to a system (e.g., a cell, organism, etc) in which a polypeptide of interest is present. In some embodiments, a host is a system that is susceptible to infection with a particular infectious agent. In some embodiments, a host is a system that expresses a particular polypeptide of interest.

*IgE binding site:* An "IgE binding site" is a region of an antigen that is recognized by an anti-antigen IgE molecule. Such a region is necessary and/or sufficient to result in (i) binding of the antigen to IgE; (ii) cross-linking of anti-antigen IgE; (iii) degranulation of mast cells containing surface-bound anti-antigen IgE; and/or (iv) development of allergic symptoms (e.g., histamine release). In general, IgE binding sites are defined for a particular antigen or antigen fragment by exposing that antigen or fragment to serum from allergic individuals (preferably of the species to whom inventive compositions are to be administered). It will be recognized that different individuals may generate IgE that recognize different epitopes on the same antigen. Thus, it is typically desirable to expose antigen or fragment to a representative pool of serum samples. For example, where it is desired that sites recognized by human IgE be identified in a given antigen or fragment, serum is preferably pooled from at least 5-10, preferably at least 15, individuals with demonstrated allergy to the antigen. Those of ordinary skill in the art will be well aware of useful pooling strategy in other contexts.

*Immunodominant:* A particular epitope is considered to be "immunodominant" if it (i) is responsible for a significant fraction of the IgE binding observed with the intact antigen; and/or (ii) is recognized by IgE in a significant fraction of sensitive individuals. An immunodominant epitope is often defined in reference to the other observed epitopes. For example, all IgE epitopes in a given antigen can be assayed simultaneously (e.g., by immunoblot) and the immunodominant epitopes can be identified by their strength as compared with the other epitopes. Usually, but not always, an immunodominant epitope will contribute at least 10% of the binding reactivity observed in such a study. Alternatively or additionally, an epitope can be classified as immunodominant if it is recognized by IgE in sera of a significant fraction, preferably at least a majority, more preferably at least about 60%, 70%, 80%, 90%, 95%, 99%, or 100%, of sensitive individuals.

*Isolated:* The term "isolated", as used herein, refers to an agent or entity that has been either (i) separated from at least some of the components with which it was associated when initially produced (whether in nature or in an experimental setting); or (ii) produced by the hand of man. Isolated agents or entities may be separated from at least about 10%, at least about 20%, at least about 30%, at least about 40%, at least about 50%, at least about 60%, at least about 70%, at least about 80%, at least about 90%, or more of the other components with which they were initially associated. In some embodiments, isolated agents are more than 90%, 91%, 92%, 93%, 94%, 95%, 96%, 97%, 98%, 99% pure.

*Long oligosaccharide:* For purposes of the present disclosure, an oligosaccharide is typically considered to be "long" if it includes at least one linear chain that has at least four saccharide residues.

*Low affinity binding:* The term "low affinity binding", as used herein refers to a low degree of tightness with which a particular ligand (e.g., an HA polypeptide) binds to its partner (e.g., an HA receptor). As described herein, affinities can be measured by any available method, including methods known in the art. In some embodiments, binding is considered to be low affinity if the Kd' is about 100 pM or more (e.g., above about 200 pM, 300 pM, 400 pM, 500 pM, 600 pM, 700 pM, 800 pM, 900 pM, InM, 1.1.nM, 1.2 nM, 1.3 nM, 1.4 nM, 1.5 nM, etc.) In some embodiments, binding is considered to be low affinity if the affinity is the same or lower (e.g., the Kd' is about the same or higher) for a polypeptide of interest than for a selected reference polypeptide. In some embodiments, binding is considered to be low affinity if the ratio of the Kd' for a polypeptide of interest to the Kd' for a selected reference polypeptide is 1:1 or more (e.g., 1.1:1, 1.2:1, 1.3:1, 1.4:1, 1.5:1. 1.6:1, 1.7:1, 1.8:1, 1.9:1, 2:1, 3:1, 4:1, 5:1, 10:1 or more). In some embodiments, binding is considered to be low affinity if the Kd' for a polypeptide of interest is 100% or more (e.g., 100%, 105%, 110%, 115%, 120%, 125%, 130%, 135%, 140%, 145%, 150%, 155%, 160%, 165%, 170%, 175%, 180%, 185%, 190%, 195%, 200%, 300%, 400%, 500%, 1000%, or more) of the Kd' for a selected reference polypeptide.

*Non-natural amino acid:* The phrase "non-natural amino acid" refers to an entity having the chemical structure of an amino acid and therefore being capable of participating in at least two peptide bonds, but having an R group that differs from those found in nature. In some embodiments, non-natural amino acids may also have a second R group rather than a hydrogen, and/or may have one or more other substitutions on the amino or carboxylic acid moieties.

*Pandemic strain:* A "pandemic" influenza strain is one that has or has capacity to cause pandemic infection of human populations. In some embodiments, a pandemic strain has caused pandemic infection. In some embodiments, such pandemic infection involves epidemic infection across multiple territories, and particularly across territories that are separated from one another (e.g., by mountains, bodies of water, as part of distinct continents, etc) such that infections ordinarily do not pass between them.

*Polypeptide:* A "polypeptide", generally speaking, is a string of at least two amino acids attached to one another by a peptide bond. In some embodiments, a polypeptide may include at least 3-5 amino acids, each of which is attached to others by way of at least one peptide bond. Those of ordinary skill in the art will appreciate that polypeptides sometimes include "non-natural" amino acids or other entities that nonetheless are capable of integrating into a polypeptide chain, optionally.

*Predominantly present:* The term "predominantly present", as used herein, refers to the presence of an entity (e.g., an amino acid residue) at a particular location across a population. For example, an amino acid may be predominantly present if, across a population of polypeptides, a particular amino acid is statistically present at a particular position in at least about 50%, about 55%, about 60%, about 65%, about 70%, about 75%, about 80%, about 85%, about 90%, about 95%, about 96%, about 97%, about 98%, about 99% or more of the polypeptides within a relevant population.

*Prevention:* The term "prevention", as used herein, refers to a delay of onset, and/or reduction in frequency and/or severity of one or more symptoms of a particular disease, disorder or condition. In some embodiments, prevention is assessed on a population basis such that an agent is considered to "prevent" a particular disease, disorder or condition if a statistically significant decrease in the development, frequency, and/or intensity of one or more symptoms of the disease, disorder or condition is observed in a population susceptible to the disease, disorder, or condition.

*Pure:* As used herein, an agent or entity is "pure" if it is substantially free of other components. For example, a preparation that contains more than about 90% of a particular agent or entity is typically considered to be a pure preparation. In some embodiments, an agent or entity is at least 91%, at least 92%, at least 93%, at least 94%, at least 95%, at least 96%, at least 97%, at least 98%, or at least 99% pure.

*Receptor-Binding Site (RBS):* As used herein, the term "receptor-binding site" or "RBS" comprises residues spanning positions 56 to 73, 87-96, 127-160 and 183-230 (numbered according to H5 HA crystal structure PDB ID: 2IBX) which include direct-binding amino acids.

*Seeding potential:* As used herein, the term "seeding potential" refers to a likelihood of an agent (e.g., an infectious agent such as a virus, a bacterium, etc.) to propagate infection. In some embodiments, seeding potential is correlated with the ability of an agent (e.g., an infectious agent such as a virus, a bacterium, etc.) to give rise to variant progeny. For example, a seed strain may have 10%, 15%, 20%, 25%, 30%, 35%, 40%, 45%, 50%, 55%, 60%, 65%, 70%, 75%, 80%, 85%, 90%, 95%, or 100% variant progeny.

*Short oligosaccharide:* For purposes of the present disclosure, an oligosaccharide is typically considered to be "short" if it has fewer than 4, or certainly fewer than 3, residues in any linear chain.

*Receptor-Binding Site Network (RBSN):* The term "receptor-binding site network (RBSN)" refers to a set of amino acid residues that are a part of the RBS that are arranged in three-dimensional space to permit interaction with one another in the context of a folded polypeptide chain. The amino acid residues include direct-binding amino acids that make contacts with glycan receptor in the *cone-like* and *umbrella-like* topology

*Receptor-Binding Site Network (RBSN) Diagram:* As used herein, the term "receptor-binding site network (RBSN) diagram" refers to a two-dimensional open connectivity network diagram that captures interactional relationship between amino acids in the RBS.

*Receptor-Binding Site Network (RBSN) Score:* As used herein, the term "receptor-binding site network (RBSN) score" refers to a score assigned to an amino acid residue in a polypeptide based on the extent of its network of interactions with other amino acids in its close spatial environment (such as the ability of that residue's side chain to interact with side chains of other residues in the polypeptide), and/or on the nature of such interactions, as described herein. For example, as described herein, the RBSN score varies from 0 (absence of any network) to 1 (most networked). The higher the network of an amino acid within the RBS, the more it is structurally constrained to be mutated.

*Specificity:* As is known in the art, "specificity" is a measure of the ability of a particular ligand (e.g., an HA polypeptide) to distinguish its binding partner (e.g., a human HA receptor, and particularly a human upper respiratory tract HA receptor) from other potential binding partners (e.g., an avian HA receptor).

*Substantial numerical similarity:* As used herein, the term "substantial numerical similarity" refers to two values, for example, two RBSN scores, having a numerical value that does not differ by more than 30%.

*Substantial sequence homology:* The phrase "substantial homology" is used herein to refer to a comparison between amino acid or nucleic acid sequences. As will be appreciated by those of ordinary skill in the art, two sequences are generally considered to be "substantially homologous" if they contain homologous residues in corresponding positions. Homologous residues may be identical residues. Alternatively, homologous residues may be non-identical residues will appropriately similar structural and/or functional characteristics. For example, as is well known by those of ordinary skill in the art, certain amino acids are typically classified as "hydrophobic" or "hydrophilic"amino acids., and/or as having "polar" or "non-polar" side chains Substitution of one amino acid for another of the same type may often be considered a "homologous" substitution. Typical amino acid categorizations are summarized below:

| Alanine | Ala | A | nonpolar | neutral | 1.8 |
|---|---|---|---|---|---|
| Arginine | Arg | R | polar | positive | -4.5 |
| Asparagine | Asn | N | polar | neutral | -3.5 |
| Aspartic acid | Asp | D | polar | negative | -3.5 |
| Cysteine | Cys | c | nonpolar | neutral | 2.5 |
| Glutamic acid | Glu | E | polar | negative | -3.5 |
| Glutamine | Gln | Q | polar | neutral | -3.5 |
| Glycine | Gly | G | nonpolar | neutral | -0.4 |
| Histidine | His | H | polar | positive | -3.2 |
| Isoleucine | Ile | I | nonpolar | neutral | 4.5 |
| Leucine | Leu | L | nonpolar | neutral | 3.8 |

(continued)

| Lysine | Lys | K | polar | positive | -3.9 |
|---|---|---|---|---|---|
| Methionine | Met | M | nonpolar | neutral | 1.9 |
| Phenylalanine | Phe | F | nonpolar | neutral | 2.8 |
| Proline | Pro | P | nonpolar | neutral | -1.6 |
| Serine | Ser | S | polar | neutral | -0.8 |
| Threonine | Thr | T | polar | neutral | -0.7 |
| Tryptophan | Trp | w | nonpolar | neutral | -0.9 |
| Tyrosine | Tyr | Y | polar | neutral | -1.3 |
| Valine | Val | V | nonpolar | neutral | 4.2 |

| Ambiguous Amino Acids | 3-Letter | 1-Letter |
|---|---|---|
| Asparagine or aspartic acid | Asx | B |
| Glutamine or glutamic acid | Glx | Z |
| Leucine or Isoleucine | Xle | J |
| Unspecified or unknown amino acid | Xaa | X |

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology. In some embodiments, two sequences are considered to be substantially homologous if at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or more of their corresponding residues are homologous over a relevant stretch of residues. In some embodiments, the relevant stretch is a complete sequence. In some embodiments, the relevant stretch is at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, at least 250, at least 275, at least 300, at least 325, at least 350, at least 375, at least 400, at least 425, at least 450, at least 475, at least 500 or more residues.

*Substantial sequence identity:* The phrase "substantial identity" is used herein to refer to a comparison between amino acid or nucleic acid sequences. As will be appreciated by those of ordinary skill in the art, two sequences are generally considered to be "substantially identical" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, et al., Basic local alignment search tool, J. Mol. Biol., 215(3): 403-410, 1990; Altschul, et al., Methods in Enzymology; Altschul, et al., "Gapped BLAST and PSI-BLAST: a new generation of protein database search programs", Nucleic Acids Res. 25:3389-3402, 1997; Baxevanis, et al., Bioinformatics : A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying identical sequences, the programs mentioned above typically provide an indication of the degree of identity. In some embodiments, two sequences are considered to be substantially identical if at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or more of their corresponding residues are identical over a relevant stretch of residues. In some embodiments, the relevant stretch is a complete sequence. In some embodiments, the relevant stretch is at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 225, at least 250, at least 275, at least 300, at least 325, at least 350, at least 375, at least 400, at least 425, at least 450, at least 475, at least 500 or more residues.

*Substantial structural similarity*: As used herein, the term "substantial structural similarity" refers to presence of shared structural features such as presence and/or identity of particular amino acids at particular positions (see definitions of "shared sequence homology" and "shared sequence identity"). In some embodiments the term "substantial structural similarity" refers to presence and/or identity of structural elements (for example: loops, sheets, helices, H-bond donors, H-bond acceptors, glycosylation patterns, salt bridges, and disulfide bonds). In some other embodiments, the term "substantial structural similarity" refers to three dimensional arrangement and/or orientation of atoms or moieties relative to one another (for example: distance and/or angles between or among them between an agent of interest and a reference agent).

*Therapeutic agent:* As used herein, the phrase "therapeutic agent" refers to any agent that elicits a desired biological or pharmacological effect.

*Treatment:* As used herein, the term "treatment" refers to any method used to alleviate, delay onset, reduce severity or incidence, or yield prophylaxis of one or more symptoms or aspects of a disease, disorder, or condition. A treatment can be administered before, during, and/or after the onset of symptoms.

*Umbrella topology:* The phrase "umbrella topology" is used herein to refer to a 3-dimensional arrangement adopted by certain glycans and in particular by glycans on HA receptors. This is in contrast to glycans having a "cone topology" as shown in FIG. 8A and 9. As described in PCT Patent Application Nos. PCT/US09/30056 and PCT/US07/18160, binding to umbrella topology glycans is characteristic of HA proteins that mediate infection of human hosts. The umbrella topology is typically adopted only by $\alpha$2-6 sialylated glycans, and is typical of long (e.g., greater than tetrasaccharide) oligosaccharides. In some embodiments, umbrella-topology glycans are glycans exhibiting a three-dimensional structure substantially similar to the structure presented in FIG. 8B. In some embodiments, umbrella-topology glycans are glycans which contact HA polypeptides via the amino acid residues shown in FIG. 8B. In some embodiments, umbrella-topology glycans are glycans which are able to contact and/or specifically bind to the amino acid binding pocket shown in FIG. 8B. In some embodiments, glycan structural topology is classified based on parameter $\theta$ defined as angle between $C_2$ of Sia, $C_1$ of Gal, and $C_1$ of GlcNAc. Values of $\theta < 100°$ represent *cone*-like topology adopted by $\alpha$2-3 and short $\alpha$2-6 glycans. Values of $\theta > 110°$ represent *umbrella*-like topology, such as topology adopted by long $\alpha$2-6 glycans. An example of umbrella topology is given by $\phi$ angle of Neu5Ac$\alpha$2-6Gal linkage of around -60. In some embodiments, umbrella-topology glycans (e.g., at a site) comprise a greater proportion of long (*e.g.* multiple lactosamine units) $\alpha$2-6 oligosaccharide branches than short $\alpha$2-6 (*e.g.* single lactosamine) branches. Exemplary N- and O-linked glycan structures capable of adopting an umbrella topology are found in FIGS. 10 and 11. In some embodiments, umbrella-topology glycans (e.g., at a site) comprise about 2-fold, about 3-fold, about 4-fold, about 5-fold, about 10-fold, about 20-fold, about 50-fold, or greater than about 50-fold more long $\alpha$2-6 oligosaccharide branches than short $\alpha$2-6 (*e.g.* single lactosamine) branches. In some embodiments, the unique characteristic of HA interactions with umbrella-topology glycans and/or glycan decoys is the HA contact with a glycan comprising sialic acid (SA) and/or SA analogs at the non-reducing end. In some embodiments, chain length of the oligosaccharide is at least a trisaccharide (excluding the SA or SA analog).

In some embodiments, umbrella topology glycans are oligosaccharides of the following form:

Neu5Ac$\alpha$2-6Sug1-Sug2-Sug3

where:

(a) Neu5Ac $\alpha$2-6 is typically (but not essentially) at the non-reducing end;

(b) Sug1:

(i) is a hexose (frequently Gal or Glc) or hexosamine (GlcNAc or GalNAc) in $\alpha$ or $\beta$ configuration (frequently $\beta$- for N- and O-linked extension and $\alpha$- in the case of GalNAc$\alpha$- that is O-linked to glycoprotein);
(ii) no sugars other than Neu5Ac$\alpha$2-6 are attached to any of the non-reducing positions of Sugl (except when Sug1 is GalNAc$\alpha$- that is O-linked to the glycoprotein); and/or
(iii) non-sugar moieties such as sulfate, phosphate, guanidium, amine, N-acetyl, *etc.* can be attached to non-reducing positions (typically 6 position) of Sug1 (e.g., to improve contacts with HA);

(c) Sug2 and/or Sug3 is/are:

(i) hexose (frequently Gal or Glc) or hexosamine (GlcNAc or GalNAc) in $\alpha$ or $\beta$ configuration (frequently $\beta$); and/or
(ii) sugars (such as Fuc) or non-sugar moieties such as sulfate, phosphate, guanidium, amine, N-acetyl, *etc.* can be attached to non-reducing positions of Sug2, Sug3, and/or Sug4;

(d) Linkage between any two sugars in the oligosaccharide apart from Neu5Ac$\alpha$2-6 linkage can be 1-2, 1-3, 1-4, and/or 1-6 (typically 1-3 or 1-4); and/or

(e) Structure where Neu5Ac$\alpha$2-6 is linked GalNAc$\alpha$ that is O-linked to the glycoprotein and additional sugars are linked to the non-reducing end of GalNAc$\alpha$ for example

(i) Neu5Ac$\alpha$2-6(Neu5Ac$\alpha$2-3Gal$\beta$1-3)GalNAc$\alpha$-

(ii) Neu5Ac$\alpha$2-6(Gal$\beta$1-3)GalNAc$\alpha$-.

By way of example only, in some embodiments, umbrella topology glycans are oligosaccharides of the following form: Neu5Ac$\alpha$2-6Gal$\beta$1-4GlcNAc$\beta$1-3Gal$\beta$1-4GlcNAc-, Neu5Ac$\alpha$2-6GalNAc$\beta$1-4GlcNAc$\beta$1-3GalNAc$\beta$1-4GlcNAc-, Neu5Ac$\alpha$2-6GlcNAc$\beta$1-3Gal$\beta$1-3/4GlcNAc-, Neu5Ac$\alpha$2-6Gal$\beta$1-4GlcNAc$\beta$1-3Gal$\beta$1-3GalNAc$\alpha$, Neu5Ac$\alpha$2-6Gal$\beta$1-4GlcNAc$\beta$1-3Gal$\beta$1-4GlcNAc$\beta$1-3Gal$\beta$1-3GalNAc$\alpha$, Neu5Ac$\alpha$2-6GalNAc$\beta$1-4GlcNAc$\beta$1-3Gal$\beta$1-3GalNAc$\alpha$, Neu5Ac$\alpha$2-6GalNAc$\beta$1-4GlcNAc$\beta$1-3GalNAc$\beta$1-4GlcNAc$\beta$1-3Gal$\beta$1-3GalNAc$\alpha$, Neu5Ac$\alpha$2-6 GalNAc$\alpha$-$\beta$1-3Gal$\alpha$2-3Neu5Ac, Neu5Ac$\alpha$2-6Gal$\beta$1-4GlcNAc$\beta$1-3/6GalNAc$\alpha$, Neu5Ac$\alpha$2-6Gal$\beta$1-4GlcNAc$\beta$1-3Gal$\beta$1-4GlcNAc$\beta$1-3/6GalNAc$\alpha$, Neu5Ac$\alpha$2-6GalNAc$\beta$1-4GlcNAc$\beta$1-3/6GalNAc$\alpha$, Neu5Ac$\alpha$2-6GalNAc$\beta$1-4GlcNAc$\beta$1-3GalNAc$\beta$1-4GlcNAc$\beta$1-3/6GalNAc$\alpha$, Neu5Ac$\alpha$2-6Gal$\beta$1-4GlcNAc$\beta$1-6GalNAc$\alpha$-$\beta$1-3Gal$\alpha$2-3Neu5Ac, Neu5Ac$\alpha$2-6Gal$\beta$1-4GlcNAc$\beta$1-3/6GalNAc$\alpha$-$\beta$1-3/6GlcNAc$\beta$1-4Gal$\alpha$2-3/6Neu5Ac, Neu5Ac$\alpha$2-6GlcNAc$\beta$1-3Gal$\beta$1-4GlcNAc$\beta$1-3Gal$\beta$1-3GalNAc, Neu5Ac$\alpha$2-6GlcNAc$\beta$1-3Gal$\beta$1-3GlcNAc$\beta$1-3/6GalNAc, Neu5Ac$\alpha$2-6GlcNAc$\beta$1-3Gal$\beta$1-4GlcNAc$\beta$1-3/6GalNAc, Neu5Ac$\alpha$2-6Gal$\beta$1-3GalNAc$\beta$1-4Gal$\alpha$1-3Gal$\beta$1-4Glc, Neu5Ac$\alpha$2-6Gal$\beta$1-4GlcNAc$\beta$1-3Gal$\beta$1-4Glc, Neu5Ac$\alpha$2-6Gal$\beta$1-3GalNAc$\beta$1-3Gal$\alpha$1-4Gal$\beta$1-4Glc, Neu5Ac$\alpha$2-6Gal$\beta$1-3GlcNAc$\beta$1 -3Gal$\beta$1-4Glc, Neu5$\alpha$2-6Gal$\beta$1-4GlcNAc$\beta$1-3GalNAc$\alpha$, Neu5Ac$\alpha$2-6Gal$\beta$1-4GlcNAc$\beta$1-3Gal$\beta$1-3GalNAc$\alpha$, Neu5Ac$\alpha$2-6GalNAc($\beta$1-3Gal-)$\beta$1-4Gal$\beta$1-4Glc, Neu5Ac$\alpha$2-6GalNAc($\beta$1-3Gal-)$\beta$1-3Gal$\alpha$1-4Gal$\beta$1-4Glc, and combinations thereof.

*Unit dose:* The expression "unit dose" as used herein refers to a physically discrete unit of a pharmaceutical composition, formulated for administration to a subject. In many embodiments, a unit dose contains a predetermined quantity of an active agent. In some embodiments, a unit dose contains an entire single dose of the agent. In some embodiments, more than one unit dose is administered to achieve a total single dose. In some embodiments, administration of multiple doses is required, or expected to be required, in order to achieve an intended effect. The unit dose may be, for example, a volume of liquid (e.g., an acceptable carrier) containing a predetermined quantity of one or more therapeutic agents, a predetermined amount of one or more therapeutic agents in solid form, a sustained release formulation or drug delivery device containing a predetermined amount of one or more therapeutic agents, etc. It will be appreciated that a unit dose may contain a variety of components in addition to the therapeutic agent(s). For example, acceptable carriers (*e.g.,* pharmaceutically acceptable carriers), diluents, stabilizers, buffers, preservatives, etc., may be included as described *infra.* It will be understood, however, that the total daily usage of a formulation of the present disclosure will often be decided by the attending physician within the scope of sound medical judgment. In some embodiments, the specific effective dose level for any particular subject or organism may depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts.

*Vaccination:* As used herein, the term "vaccination" refers to the administration of a composition intended to generate an immune response, for example to a disease-causing agent. Vaccination can be administered before, during, and/or after exposure to a disease-causing agent, and/or to the development of one or more symptoms, and in some embodiments, before, during, and/or shortly after exposure to the agent. In some embodiments, vaccination includes multiple administrations, appropriately spaced in time, of a vaccinating composition.

*Variant:* As used herein, the term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a small molecule may have a characteristic core structural element (e.g., a macrocycle core) and/or one or more characteristic pendent moieties so that a variant of the small molecule is one that shares the core structural element and the characteristic pendent moieties but differs in other pendent moieties and/or in types of bonds present (single vs double, E vs Z, etc) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to on another in linear or three-dimensional space. For example, a variant polypeptide may differ from a reference polypeptide as a result of one or more differences in amino acid sequence and/or one or more differences in chemical moieties (e.g., carbohydrates, lipids, etc) covalently attached to the polypeptide backbone. In some embodiments, a variant polypeptide shows an overall sequence identity with a reference polypeptide that is at least 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide. In some embodiments, a variant polypeptide lacks one or more of the biological activities of the reference polypeptide. In some embodiments, a variant polypeptide shows a reduced level of one or more biological activities as compared with the reference polypeptide. In many embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (e.g., fewer than 5, 4, 3, 2, or 1) number of substituted functional residues (i.e., residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature. As will be understood by those of ordinary skill in the art, a plurality of variants of a particular polypeptide of interest may commonly be found in nature, particularly when the polypeptide of interest is an infectious agent polypeptide.

*Very Resistant-to-Evolution Cluster (VREC):* As used herein, the terms "Very Resistant-to-Evolution Cluster" and "VREC" refer to a cluster of amino acid residues that demonstrate high RBSN scores and/or low tolerance for mutation. That is, in some embodiments, a VREC cluster is one for which mutations typically result in disruption of polypeptide structure and/or function. In some embodiments, VREC cluster residues do not have positive BLOSUM62 substitution scores.

## Detailed Description of Certain Embodiments

The present invention provides, among other things, methods and compositions relating to detection, treatment, and/or prevention of influenza transmission and/or infection.

*Influenza Infection*

Influenza has a long history of pandemics, epidemics, resurgences and outbreaks. Avian influenza, including the H5N1 strain, is a highly contagious and potentially fatal pathogen, but it currently has only a limited ability to infect humans.

Influenza viruses are RNA viruses which are characterized by a lipid membrane envelope containing two glycoproteins, hemagglutinin (HA) and neuraminidase (NA), embedded in the membrane of the virus particle. The viral genome is made up of several negative sense single stranded RNA molecules. Several proteins are encoded by the viral genome. Neuraminidase (NA) is a viral surface glycoprotein that cleaves terminal sialic acid residues from carbohydrate moieties on the surfaces of infected cells, promoting the release of progeny viruses. Hemagglutinin (HA) is one of the major viral surface glycoproteins and involved in the binding of the virus to sialic acids on the surface of susceptible cells (Uiprasertkul et al. Emerg. Infect. Dis. 11:1036, 2005).

Influenza HA is a trimer on virus particles. Influenza HA is synthesized as HA0 by virus post-infection in cells that is cleaved by cellular proteases at the basic cleavage site into HA1 and HA2 mature forms, which is required for proper function of this surface protein and for viral life cycle. The M2 protein is an ion channel protein. The HA, NA, and M2 protein are present in the viral envelope which is derived from the host cell plasma membrane. A ribonucleoprotein complex comprises an RNA segment associated with nucleoprotein (NP) and three polymerases, PA, PB1, and PB2. The M1 protein is associated with both ribonucleoprotien and the envelope.

Annual epidemics of influenza occur when the antigenic properties of the viral HA and NA proteins are altered. The mechanism of altered antigenicity is twofold: antigenic shift, caused by genetic rearrangement between human and animal viruses after double infection of host cells, which can cause a pandemic; and antigenic drift, caused by small changes in the HA and NA proteins on the virus surface, which can cause influenza epidemics.

There are 16 known HA subtypes and 9 NA subtypes, and different influenza strains are named based on the number of the strain's HA and NA subtypes. Based on comparisons of amino acid sequence identity and of crystal structures, the HA subtypes have been divided into two main groups and four smaller clades. The different HA subtypes do not necessarily share strong amino acid sequence identity, but the overall 3D structures of the different HA subtypes are similar to one another, with several subtle differences that can be used for classification purposes. For example, the particular orientation of the membrane-distal subdomains in relation to a central $\alpha$-helix is one structural characteristic commonly used to determine HA subtype (Russell et al., 2004 Virology, 325:287, 2004). Those skilled in the art are well familiar with sequence and other structural similarities and differences that can be used to define and/or to distinguish different subtypes and/or clades of influenza viruses.

Only three (HI, H2, and H3) of the sixteen HA subtypes have thus far become adapted for human infection. One reported characteristic of HAs that have adapted to infect humans (e.g., of HAs from the pandemic H1N1 (1918) and H3N2 (1967-68) influenza subtypes) is their ability to preferentially bind to $\alpha$2-6 sialylated glycans in comparison with their avian progenitors that preferentially bind to $\alpha$2-3 sialylated glycans (Skehel & Wiley, 2000 Annu Rev Biochem, 69:531; Rogers, & Paulson, 1983 Virology, 127:361; Rogers et al., 1983 Nature, 304:76; Sauter et al., 1992 Biochemistry, 31:9609; Connor et al., 1994 Virology, 205:17; Tumpey et al., 2005 Science, 310:77).

Several crystal structures of HAs from H1 (human and swine), H2 (human and avian), H3 (avian) and H5 (avian) subtypes bound to sialylated oligosaccharides (of both $\alpha$2-3 and $\alpha$2-6 linkages) are available and provide molecular insights into the specific amino acids that are involved in distinct interactions of the HAs with these glycans (Eisen et al., 1997 Virology, 232:19; Ha et al., 2001 Proc Natl Acad Sci USA, 98:11181; Ha et al., 2003 Virology, 309:209; Gamblin et al., 2004 Science, 303:1838; Stevens et al., 2004 Science, 303:1866; Russell et al., 2006 Glycoconj J 23:85; Stevens et al., 2006 Science, 312:404; Xu R et al., 2010 J Virol 84(4):1715; Liu J, et al., 2009 Proc Natl Acad Sci U S A 106(40):17175).

Influenza infection is mediated by interaction of HA with the surface of cells through binding to a glycoprotein receptor. Binding of HA to HA receptors is predominantly mediated by N-linked glycans on the HA receptors. Specifically, HA on the surface of flu virus particles recognizes sialylated glycans that are associated with HA receptors on the surface of the cellular host. After recognition and binding, the host cell engulfs the viral cell and the virus is able to replicate and produce many more virus particles to be distributed to neighboring cells. Some crystal structures of exemplary HA-glycan interactions have been identified and are presented in Table 1:

**Table 1. Crystal Structures of HA-Glycan Complexes**

| Abbreviation (PDB ID) | Virus Strain | Glycan (with assigned coordinates) |
|---|---|---|
| ASI30_H1_23 (1RV0) | A/Swine/Iowa/30 (H1N1) | Neu5Ac |
| ASI30_H1_26 (1RVT) | A/Swine/Iowa/30 (H1N1) | Neu5Ac$\alpha$6Gal$\beta$4GlcNAc$\beta$3Gal$\beta$4Glc |
| APR34_H1_23 (1RVX) | A/Puerto Rico/8/34 (H1N1) | Neu5Ac$\alpha$3Gal$\beta$4GlcNAc |
| APR34_H1_26 (1RVZ) | A/Puerto Rico/8/34 (H1N1) | Neu5Ac$\alpha$6Gal$\beta$4GlcNAc |
| ADU63_H3_23 (1MQM) | A/Duck/Ukraine/1/63 (H3N8) | Neu5Ac$\alpha$3Gal |
| ADU63_H3_26 (1MQN) | A/Duck/Ukraine/1/63 (H3N8) | Neu5Ac$\alpha$6Gal |
| AAI68_H3_23 (1HGG) | A/Aichi/2/68 (H3N2) | Neu5Ac$\alpha$3Gal$\beta$4Glc |
| ADS97_H5_23 (1JSN) | A/Duck/Singapore/3/97 (H5N3) | Neu5Ac$\alpha$3Gal$\beta$3GlcNAc |
| ADS97_H5 _26(1JSO) | A/Duck/Singapore/3/97 (H5N3) | Neu5Ac |
| Viet04_H5 (2FK0) | A/Vietnam/1203/2004 (H5N1) | |
| HA - $\alpha$2-6 sialylated glycan complexes were generated by superimposition of the CA trace of the HA1 subunit of ADU63H3 and ADS97H5 and Viet04_H5 on ASI30_H1_26 and APR34_H1_26 (HI). Although the structural complexes of the human A/Aichi/2/68 (H3N2) with $\alpha$2-6 sialylated glycans are published (Eisen *et al.,* 1997, *Virology,* 232:19), their coordinates were not available in the Protein Data Bank. The SARF2 (http://123d.ncifcrf.gov/sarf2.html) program was used to obtain the structural alignment of the different HA1 subunits for superimposition. | | |

HA receptors are modified by either $\alpha$2-3 or $\alpha$2-6 sialylated glycans near the receptor's HA-binding site, and the type of linkage of the receptor-bound glycan can affect the conformation of the receptor's HA-binding site, thus affecting the receptor's specificity for different HAs.

For example, the glycan binding pocket of avian HA is narrow. According to the present invention, this pocket binds to the *trans* conformation of $\alpha$2-3 sialylated glycans, and/or to cone-topology glycans, whether $\alpha$2-3 or $\alpha$2-6 linked.

HA receptors in avian tissues, and also in human deep lung and gastrointestinal (GI) tract tissues are characterized by $\alpha$2-3 sialylated glycan linkages, and furthermore (according to the present invention), are characterized by glycans, including $\alpha$2-3 sialylated and/or $\alpha$2-6 sialylated glycans, which predominantly adopt cone topologies. HA receptors having such cone-topology glycans may be referred to herein as CTHArs.

By contrast, human HA receptors in the bronchus and trachea of the upper respiratory tract are modified by glycans which predominantly adopt umbrella topologies, for example including many $\alpha$2-6 sialylated glycans. Unlike the $\alpha$2-3 motif, the $\alpha$2-6 motif has an additional degree of conformational freedom due to the C6-C5 bond (Russell et al., Glycoconj J 23:85, 2006). HAs that bind to such $\alpha$2-6 sialylated glycans have a more open binding pocket to accommodate the diversity of structures arising from this conformational freedom. Moreover, as described in PCT Patent Application Nos. PCT/US09/30056 and PCT/US07/18160, HAs may need to bind to glycans (e.g., $\alpha$2-6 sialylated glycans) in an umbrella topology, and particularly may need to bind to such umbrella topology glycans with strong affinity and/or specificity, in order to effectively mediate infection of human upper respiratory tract tissues. HA receptors having umbrella-topology glycans may be referred to herein as UTHArs.

As a result of these spatially restricted glycosylation profiles, humans are not usually infected by viruses containing many wild type avian HAs (e.g., avian H5). Specifically, because the portions of the human respiratory tract that are most likely to encounter virus (i.e., the trachea and bronchi) lack receptors with cone glycans (e.g., $\alpha$2-3 sialylated glycans, and/or short glycans) and wild type avian HAs typically bind primarily or exclusively to receptors associated with cone glycans (e.g., $\alpha$2-3 sialylated glycans, and/or short glycans), humans rarely become infected with avian viruses. Only when in sufficiently close contact with virus that it can access the deep lung and/or gastrointestinal tract receptors having umbrella glycans (e.g., long $\alpha$2-6 sialylated glycans) do humans become infected.

*HA Polypeptides*

The present disclosure defines and describes certain HA polypeptides, specifically including H5 HA polypeptides that show overall sequence identity with a reference HA and also include particular structural features as described herein. The present disclosure also provides fragments of such HA polypeptides, including characteristic fragments (i.e., fragments whose amino acid sequence includes at least one characteristic sequence element). In some embodiments, provided HA polypeptides mediate significant human receptor binding and/or human infection and/or transmission (e.g., as assessed in an established or described assay system).

In some embodiments, provided HA polypeptides bind to umbrella topology glycans (e.g., long $\alpha$2-6 sialylated glycans such as, for example, Neu5Ac$\alpha$2-6Gal$\beta$1-4GlcNAc$\beta$1-3Gal$\beta$1-4GlcNAc-) with high affinity. For example, in some embodiments, provided HA polypeptides bind to umbrella topology glycans with an affinity comparable to that observed for a wild type HA that mediates infection of a humans (e.g., H1N1 HA or H3N2 HA). In some embodiments, provided HA polypeptides bind to umbrella glycans with an affinity that is at least 25%, 30%, 35%, 40%, 45%, 50%, 55%, 60%, 65%, 70%, 75%, 80%, 85%, 90%, 95%, 96%, 97%, 98%, 99%, or 100% of that observed under comparable conditions for a wild type HA that mediates infection of humans. In some embodiments, provided HA polypeptides bind to umbrella glycans with an affinity that is greater than that observed under comparable conditions for a wild type HA that mediates infection of humans.

In certain embodiments, binding affinity of provided HA polypeptides is assessed over a range of concentrations. Such a strategy provides significantly more information, particularly in multivalent binding assays, than do single-concentration analyses. In some embodiments, for example, binding affinities of provided HA polypeptides are assessed over concentrations ranging over at least 2, 3, 4, 5, 6, 7, 8, 9, 10 or more fold.

In certain embodiments, provided HA polypeptides show high affinity if they show a saturating signal in a multivalent glycan array binding assay such as those described herein. In some embodiments, provided HA polypeptides show high affinity if they show a signal above about 400000 or more (e.g., above about 500000, 600000, 700000, 800000, etc) in such studies. In some embodiments, binding agents as described herein show saturating binding to umbrella glycans over a concentration range of at least 2 fold, 3 fold, 4 fold, 5 fold or more, and in some embodiments over a concentration range as large as 10 fold or more.

Furthermore, in some embodiments, provided HA polypeptides bind to umbrella topology glycans (and/or to umbrella topology glycan mimics) more strongly than they bind to cone topology glycans. In some embodiments, provided HA polypeptides show a relative affinity for umbrella glycans vs cone glycans that is about 10, 9, 8, 7, 6, 5, 4, 3, or 2.

In some embodiments, provided HA polypeptides bind to $\alpha$2-6 sialylated glycans; in some embodiments, provided HA polypeptides bind preferentially to $\alpha$2-6 sialylated glycans. In certain embodiments, provided HA polypeptides bind to a plurality of different $\alpha$2-6 sialylated glycans. In some embodiments, provided HA polypeptides are not able to

bind to $\alpha$2-3 sialylated glycans, and in other embodiments provided HA polypeptides are able to bind to $\alpha$2-3 sialylated glycans.

In some embodiments, provided HA polypeptides bind to receptors found on human upper respiratory epithelial cells. In certain embodiments, provided HA polypeptides bind to HA receptors in the bronchus and/or trachea. In some embodiments, provided HA polypeptides are not able to bind receptors in the deep lung, and in other embodiments, provided HA polypeptides are able to bind receptors in the deep lung.

In some embodiments, provided HA polypeptides bind to at least about 10%, 15%, 20%, 25%, 30% 35%, 40%, 45%, 50%, 55%, 60%, 65%, 70%, 75%, 80%, 85%, 90% 95% or more of the glycans found on HA receptors in human upper respiratory tract tissues (e.g., epithelial cells).

In some embodiments, provided HA polypeptides are characterized in that they bind to a receptor binding site utilized by a pandemic strain of influenza, and in some embodiments compete with such pandemic strain (or a receptor-binding portion thereof), for binding to such site. In some embodiments, provided HA polypeptides are characterized by substantial numerical similarity between their RBSN score and that of an HA polypeptide found in a pandemic influenza strain.

In some embodiments, provided HA polypeptides display an activity of interest (e.g., binding to umbrella-topology glycans, mediating human infectivity and/or transmissibility, etc) for example binding to umbrella-topology glycans as measured using the glycan array analysis described here where Kd' is in the range of sub-picomolar to 10 nanomolar and at a level relative to binding to cone-topology glycans of greater than 2 orders of magnitude; in some embodiments, such relative level is relative to a different activity of the same HA polypeptide (e.g., bining to cone-topology glycans, mediating non-human infectivity and/or transmissibility, etc). In some embodiments, such relative level is relative to the same activity of a different HA polypeptide (e.g., by a reference HA).

In some embodiments, provided HA polypeptides are variants of a parent or reference HA. In some such embodiments, provided HA polypeptides have amino acid sequences that differ from that of the parent or reference HA in the presence vs absence of one or more of the features described herein. In some embodiments, provided HA polypeptides have amino acid sequences that differ from that of the parent or reference HA in the presence vs absence of only one of the features described herein. In some embodiments, provided HA polypeptides have amino acid sequences that differ from that of the parent or reference HA in the presence vs absence of 1, 2, 3, or 4 of the features described herein.

In some embodiments, the reference HA with which a provided HA polypeptide shows the specified degree of sequence identity is one that does not mediate significant human receptor binding and/or human infection and/or transmission; in some such embodiments, the provided HA differs from the reference non-human-infecting HA both in the presence vs absence of one or more structural features as described herein and in ability to mediate significant human receptor binding and/or significant human infection and/or transmission. In some embodiments, the reference HA with which a provided HA polypeptide shows the specified degree of sequence identity does mediate significant human receptor binding and/or significant human infection and/or transmission; in some such embodiments, the provided HA polypeptide shares both one or more structural features as describe herein and one or more biological activities (e.g., ability to mediate significant human receptor binding and/or significant human infection and/or transmission) with the human-infecting reference HA.

Representative HAs that do not mediate significant human receptor binding and/or human infection and/or transmission (i.e., non-human-infecting HAs) include H5 HAs, for example, A/duck/Hunan/795/2002 (clade 2.1), A/Viet Nam/1194/2004 (clade 1), A/Indonesia/5/2005 (clade 2.1.3.2), A/bar-headed goose/Qinghai/1A/2005 (clade 2.2), A/Anhui/1/2005 (clade 2.3.4), A/goose/Guiyang/337/2006 (clade 4), A/Cambodia/R0405050/2007 (clade 1.1), A/common magpie/Hong Kong/5052/2007 (clade 2.3.2.1), A/chicken/Viet Nam/NCVD-016/2008 (clade 7.1), A/Egypt/N03072/2010 (clade 2.2.1), A/Hubei/1/2010 (clade 2.3.2.1)

Representative HAs that do mediate significant human receptor binding and/or human infection and/or transmission (i.e., human-infecting HAs) including, for example **H3N2** strains including, but not limited to, A/Port Chalmers/1/1973 (H3N2), A/Scotland/840/74 (H3N2), A/Victoria/3/75(H3N2), A/Texas/1/77(H3N2), A/Bangkok/01/1979(H3N2), A/Philippines/2/82(H3N2), A/Christchurch/4/1985(H3N2), A/Mississippi/1/85(H3N2), A/Leningrad/360/1986(H3N2), A/Shanghai/11/87(H3N2), A/Sichuan/02/87(H3N2), A/Beijing/353/89(H3N2), A/Guizhou/54/89(H3N2), A/Beijing/32/92(H3N2), A/Shangdong/9/93(H3N2), A/Johannesburg/33/94(H3N2), A/Wuhan/359/95(H3N2), A/Sydney/5/97(H3N2), A/Moscow/10/99(H3N2), A/Fujian/411/2002(H3N2), A/California/7/2004(H3N2), A/Wellington/1/2004(H3N2), A/Brisbane/10/2007(H3N2), A/Perth/16/2009(H3N2), and A/Victoria/361/2011(H3N2), **H1N1** stains including, but not limited to, A/Chile/1/83(H1N1), A/Singapore/6/1986(H1N1), A/Bayern/7/95(H1N1), A/Beijing/262/95(H1N1), A/New Caledonia/20/1999(H1N1), A/Solomon Islands/3/2006(H1N1), A/Brisbane/59/2007(H1N1), and A/California/07/2009(H1N1), **H2N2** strains including, but not limited to, A/Panama/1/66(H2N2), and A/Korea/426/1968(H2N2), and, in certain cases, **H9N2** strains including, but not limited to A/guinea fowl/Hong Kong/WF10/99(H9N2), A/wild duck/Nanchang/2-0480/2000(H9N2), A/turkey/Israel/689/2008(H9N2), A/chicken/Zhejiang/HE1/2009(H9N2), and A/chicken/Egypt/115617V/2011(H9N2).

In some embodiments, the present invention provides a novel framework to define amino acid mutations in the hemagglutinin (HA) of circulating avian influenza strains, that could result in a quantitative switch in binding preference to human glycan receptors. In some embodiments, the present invention provides a novel framework to analyze molecular features of glycan receptor-binding site (RBS) of a candidate influenza HA in relation to its nearest human-adapted phylogenetic relative pandemic influenza HA. In some embodiments, the present invention demonstrates that currently circulating candidate influenza HAs have evolved such that their RBS molecular features resemble those of pandemic influenza HAs and require fewer amino acid changes to switch receptor specificity. Application of such provided frameworks defines HA polypeptide variants having sequence features and activities as described herein.

In particular, the present disclosure describes four structural features that, when present in an H5 HA polypeptide as described herein, result in a significant level of one or more activities selected from the group consisting of human receptor binding, human infection and/or human transmission. In some embodiments, an activity is considered significant if it is observed at a level above a designated threshold. In some embodiments, an activity is considered significant if it is observed at a level relatively higher than a reference activity - such as the same activity in a comparable reference HA polypeptide, for example that lacks one or more particular sequence elements or features, or as a different activity by the same HA polypeptide (e.g., binding to a different target).

As described herein, the present disclosure defines at least four structural features that contribute to relevant activities of H5 HA polypeptides. In particular, in accordance with the present disclosure provided H5 HA polypeptides typically show at least 80%, 81%, 82%, 83%, 84%, 85%, 86%, 87%, 88%, 89%, 90%, 91%, 92%, 93%, 94%, 95%, 96%, 97%, 98%, 99% or greater overall sequence identity with a reference HA (e.g., with a reference H5 HA), but have a sequence that is not 100% identical to the reference HA in that the provided HA has an amino acid sequence that includes at least one of:

1) A first feature that is a deletion of the amino acid corresponding to amino acid 130 of the reference H5 HA;

2) A second feature that is or comprises

i. $Xaa_{226}$ + ser228,
ii. $Lys_{224}$ + $Xaa_{226}$,
iii. $Xaa_{137}$ + $Xaa_{226}$ + ser228,
iv. Xaa1 + gly227 + ser228,
v. $Xaa_{137}$ + pro221 + $Xaa_{226}$ + ser228, and
vi. $Xaa_{137}$ + thr155 + pro221 + $Xaa_{226}$ + gly227 + ser228;

wherein $Xaa_{226}$ is selected from the group Leu, Ile, Val, Met, and Ala, $Xaa_{137}$ is selected from the group Arg, Lys, Gln, Glu, His, and Asn;

3) A third feature that is or comprises

i. glu188 + $Xaa_{192}$ + $Xaa_{193}$,
ii. asp187 + $Xaa_{193}$, and
iii. $Xaa_{193}$; and

wherein $Xaa_{192}$ is selected from the group Arg, Thr, Ala, Val, Leu, and Ile, and $Xaa_{193}$ is selected from the group Thr, Ala, Lys, Arg, and His; and

4) a further feature that is or comprises

i. ala160,
ii. asn158 + ala160, and
iii. asn158 + thr160,

wherein the position of the amino acids of the second, third, and fourth features correspond to the referenced position of the reference H5 HA.

*Nucleic Acids and Expression Systems*

The present disclosure also provides nucleic acids that encode polypeptides described herein, including for example HA polypeptides, antibodies, etc, and/or fragments thereof. The present disclosure also provides nucleic acids that are complementary to and/or hybridize with such encoding nucleic acids.

In some embodiments, provided nucleic acids are single stranded; in some embodiments they are double-stranded.

In some embodiments, provided nucleic acids have sequences and lengths, as will be appreciated by those skilled in the art, appropriate for their use as primers, probes, aptamers, siRNAs, antisense, etc). To give but a few examples, such nucleic acids can be used as primers in polymerase chain reaction (PCR), as probes for hybridization (including *in situ* hybridization), and/or as primers for reverse transcription-PCR (RT-PCR).

In certain embodiments, nucleic acids can be or comprise DNA and/or RNA. In some embodiments, inventive nucleic acids may include one or more non-natural nucleotides; in other embodiments, inventive nucleic acids include only natural nucleotides.

The present disclosure also provides expression systems, including *in vitro* systems, cell systems, and organisms that produce provided polypeptides, and/or fragments thereof

*Detecting Agents*

The present invention provides agents that detect (e.g., via direct or indirect binding) provided HA polypeptides.

In some embodiments, provided detecting agents bind, directly or indirectly, to one or more provided HA polypeptides. In some embodiments, provided detecting agents bind specifically to one or more provided HA polypeptides. In some embodiments, provided detecting agents distinguish between a provided HA polypeptide and a reference HA polypeptide with which the provided HA polypeptide shows a specified degree of overall sequence identity as described herein and/or that has an amino acid sequence that differs from that of the provided HA polypeptide with respect to one or more of the features described herein.

In some particular embodiments, provided detecting agents are antibodies or antibody-like entities that bind to provided HA polypeptides. In some embodiments, such antibodies or antibody-like entities bind specifically to provided HA polypeptides. In some embodiments, provided antibodies or antibody-like entities discriminate between provided HA polypeptides and their cognate reference HAs. In some embodiments, provided antibodies or antibody-like entities discriminate between HA polypeptides and otherwise identical HAs that differ only in presence or absence of one or more of the features specifically set forth therein.

Antibodies

In some embodiments, antibodies or antibody-like entities that bind to provided HA polypeptides do so in a manner that interferes with binding between such HA polypeptides and an HA receptor, such that levels of observed binding are reduced when the antibody or antibody-like entities are present as compared with when they are absent. In some embodiments, antibodies or antibody-like entities that bind to provided HA polypeptides do so in a manner that does not significantly interfere with binding between such HA polypeptides and an HA receptor.

Suitable antibodies that bind to provided HA polypeptides and are useful in accordance with the present invention include, but are not limited to, human antibodies, primatized antibodies, chimeric antibodies, bi-specific antibodies, humanized antibodies, conjugated antibodies (*i.e.,* antibodies conjugated or fused to other proteins, radiolabels, cytotoxins), Small Modular ImmunoPharmaceuticals ("SMIPs™"), single chain antibodies, cameloid antibodies, and antibody fragments. As used herein, the term "antibodies" also includes intact monoclonal antibodies, polyclonal antibodies, single domain antibodies (e.g., shark single domain antibodies (e.g., IgNAR or fragments thereof)), multispecific antibodies (*e.g.* bi-specific antibodies) formed from at least two intact antibodies, and antibody fragments so long as they exhibit the desired biological activity. Antibody polypeptides for use herein may be of any type (e.g., IgA, IgD, IgE, IgG, IgM).

As used herein, an "antibody fragment" includes a portion of an intact antibody, such as, for example, the antigen-binding or variable region of an antibody. Examples of antibody fragments include Fab, Fab', F(ab')2, and Fv fragments; triabodies; tetrabodies; linear antibodies; single-chain antibody molecules; and multi specific antibodies formed from antibody fragments. The term "antibody fragment" also includes any synthetic or genetically engineered protein that acts like an antibody by binding to a specific antigen to form a complex. For example, antibody fragments include isolated fragments, "Fv" fragments, consisting of the variable regions of the heavy and light chains, recombinant single chain polypeptide molecules in which light and heavy chain variable regions are connected by a peptide linker ("ScFv proteins"), and minimal recognition units consisting of the amino acid residues that mimic the hypervariable region.

Antibodies can be generated using methods well known in the art. For example, protocols for antibody production are described by Harlow and Lane, Antibodies: A Laboratory Manual, (1988). Typically, antibodies can be generated in mouse, rat, guinea pig, hamster, camel, llama, shark, or other appropriate host. Alternatively, antibodies may be made in chickens, producing IgY molecules (Schade et al., (1996) ALTEX 13(5):80-85). In some embodiments, antibodies suitable for the present invention are subhuman primate antibodies. For example, general techniques for raising therapeutically useful antibodies in baboons may be found, for example, in Goldenberg et al., international patent publication No. WO 91/11465 (1991), and in Losman et al., Int. J. Cancer 46: 310 (1990). In some embodiments, monoclonal antibodies may be prepared using hybridoma methods (Milstein and Cuello, (1983) Nature 305(5934):537-40.). In some embodiments, monoclonal antibodies may also be made by recombinant methods (U.S. Pat. No. 4,166,452, 1979).

In some embodiments, antibodies suitable for the invention may include humanized or human antibodies. Humanized forms of non-human antibodies are chimeric Igs, Ig chains or fragments (such as Fv, Fab, Fab', F(ab')2 or other antigen-binding subsequences of Abs) that contain minimal sequence derived from non-human Ig. Generally, a humanized antibody has one or more amino acid residues introduced from a non-human source. These non-human amino acid residues are often referred to as "import" residues, which are typically taken from an "import" variable domain. Humanization is accomplished by substituting rodent complementarity determining regions (CDRs) or CDR sequences for the corresponding sequences of a human antibody (Riechmann et al., Nature 332(6162):323-7, 1988; Verhoeyen et al., Science. 239(4847):1534-6, 1988.). Such "humanized" antibodies are chimeric Abs (U.S. Pat. No. 4,816,567, 1989), wherein substantially less than an intact human variable domain has been substituted by the corresponding sequence from a non-human species. In some embodiments, humanized antibodies are typically human antibodies in which some CDR residues and possibly some FR residues are substituted by residues from analogous sites in rodent Abs. Humanized antibodies include human Igs (recipient antibody) in which residues from a CDR of the recipient are replaced by residues from a CDR of a non-human species (donor antibody) such as mouse, rat or rabbit, having the desired specificity, affinity and capacity. In some instances, corresponding non-human residues replace Fv framework residues of the human Ig. Humanized antibodies may comprise residues that are found neither in the recipient antibody nor in the imported CDR or framework sequences. In general, the humanized antibody comprises substantially all of at least one, and typically two, variable domains, in which most if not all of the CDR regions correspond to those of a non-human Ig and most if not all of the FR regions are those of a human Ig consensus sequence. The humanized antibody optimally also comprises at least a portion of an Ig constant region (Fc), typically that of a human Ig (Riechmann et al., Nature 332(6162):323-7, 1988; Verhoeyen et al., Science. 239(4847): 1534-6, 1988.).

Human antibodies can also be produced using various techniques, including phage display libraries (Hoogenboom et al., Mol Immunol. (1991) 28(9): 1027-37; Marks et al., J Mol Biol. (1991) 222(3):581-97) and the preparation of human monoclonal antibodies (Reisfeld and Sell, 1985, Cancer Surv. 4(1):271-90). Similarly, introducing human Ig genes into transgenic animals in which the endogenous Ig genes have been partially or completely inactivated can be exploited to synthesize human antibodies. Upon challenge, human antibody production is observed, which closely resembles that seen in humans in all respects, including gene rearrangement, assembly, and antibody repertoire (Fishwild et al., High-avidity human IgG kappa monoclonal antibodies from a novel strain of minilocus transgenic mice, Nat Biotechnol. 1996 July; 14(7):845-51; Lonberg et al., Antigen-specific human antibodies from mice comprising four distinct genetic modifications, Nature 1994 April 28;368(6474):856-9; Lonberg and Huszar, Human antibodies from transgenic mice, Int. Rev. Immunol. 1995;13(1):65-93; Marks et al., By-passing immunization: building high affinity human antibodies by chain shuffling. Biotechnology (N Y). 1992 July; 10(7):779-83).

Aptamers

In some embodiments, provided detecting agents are aptamers. Aptamers are macromolecules composed of nucleic acid (e.g., RNA, DNA) that bind tightly to a specific molecular target (e.g., an umbrella topology glycan). A particular aptamer may be described by a linear nucleotide sequence and is typically about 15-60 nucleotides in length. Without wishing to be bound by any theory, it is contemplated that the chain of nucleotides in an aptamer form intramolecular interactions that fold the molecule into a complex three-dimensional shape, and this three-dimensional shape allows the aptamer to bind tightly to the surface of its target molecule. Given the extraordinary diversity of molecular shapes that exist within the universe of all possible nucleotide sequences, aptamers may be obtained for a wide array of molecular targets, including proteins and small molecules. In addition to high specificity, aptamers have very high affinities for their targets (e.g., affinities in the picomolar to low nanomolar range for proteins). Aptamers are chemically stable and can be boiled or frozen without loss of activity. Because they are synthetic molecules, they are amenable to a variety of modifications, which can optimize their function for particular applications. For example, aptamers can be modified to dramatically reduce their sensitivity to degradation by enzymes in the blood for use in in vivo applications. In addition, aptamers can be modified to alter their biodistribution or plasma residence time.

Identification and/or characterization of aptamers that bind (directly or indirectly and/or specifically) to provided HA polypeptides can be achieved through any of a variety of approaches, as will be appreciated by those of ordinary skill in the art.

For example, aptamers can be selected using the SELEX (Systematic Evolution of Ligands by Exponential Enrichment) method (Tuerk et al, Science 249:505, 1990). Typically, SELEX methodology involves providing a large (e.g., $10^{15}$ different molecules) library of nucleic acid molecules that is contacted with the target entity (e.g., a provided HA polypeptide or fragment thereof). The entity is contacted with members of the library for a time and under conditions sufficient to permit interaction (e.g., specific interaction) to occur.

Any of a variety of technologies is known in the art to physically isolate aptamers that interact with the target entity and/or to amplify them. In some embodiments, such isolated aptamers, which may represent a new library, enriched for members that bind to the target entity of interest, may be re-screened to further identify bindng aptamers, for example that show a particular level of affinity and/or specificity.

Typically, after about 5-15 cycles of iterative selection, partitioning and amplification process, the library is reduced to a small number of aptamers that bind tightly to the target molecule. Individual aptamer molecules can be isolated, their nucleotide sequences determined, and their properties with respect to binding affinity and/or specificity measured and/or compared.

Isolated aptamers can then be refined, for example, to eliminate nucleotides that do not contribute to target binding and/or aptamer structure, thereby producing aptamers truncated to their core binding domain. See, for example, Jayasena, Clin. Chem. 45:1628, 1999 for review of aptamer technology.

*Competing Agents*

The present disclosure provides systems for identifying and/or characterizing agents that compete with provided HA polypeptides for binding to HA receptors, and particularly to human HA receptors. Such agents may be useful, for example, in the treatment or prevention of infection mediated by an HA polypeptide having one or more structural features as described herein. In some embodiments, detecting agents as described above (including, for example, antibodies and/or aptamers) are also competing agents. In some embodiments, competing agents are not detecting agents and/or do not bind directly or indirectly to provided HA polypeptides.

*Compositions*

The present disclosure provides compositions that include as an active agent provided HA polypeptides and fragments thereof, nucleic acids that encode them, expression systems that produce them, detecting agents that detect them, and/or competing agents that compete their interactions with one or more HA receptors.

Diagnostic and Surveillance Compositions

The present invention provides a variety of compositions useful in the detection, identification, and/or characterization of influenza viruses and/or infectious. In particular embodiments, the invention provides compositions comprising detecting agents, which compositions can be contacted with clinical, pathological, or environmental samples in order to asses, for example, presence or level of a particular influenza strain, extent or progress of an influenza infection, etc.

In certain embodiments, the invention provides compositions and/or kits that specifically detect HA polypeptides as described herein with particular glycan binding and/or infectivity characteristics. Such compositions or kits may include detecting agents, for example such as antibodies that specifically recognize certain HA polypeptides (e.g., that bind to umbrella glycans and/or to $\alpha$2-6 sialylated glycans and/or to long $\alpha$2-6 sialylated glycans), which can be used to specifically detect such HA polypeptides, for example by ELISA, immunofluorescence, and/or immunoblotting.

Antibodies that bind to HA polypeptides can also be used in virus neutralization tests, in which a sample is treated with antibody specific to HA polypeptides of interest, and tested for its ability to infect cultured cells relative to untreated sample. If the virus in that sample contains such HA polypeptides, the antibody will neutralize the virus and prevent it from infecting the cultured cells. Alternatively or additionally, such antibodies can also be used in HA-inhibition tests, in which the HA protein is isolated from a given sample, treated with antibody specific to a particular HA polypeptide or set of HA polypeptides, and tested for its ability to agglutinate erythrocytes relative to untreated sample. If the virus in the sample contains such an HA polypeptide, the antibody will neutralize the activity of the HA polypeptide and prevent it from agglutinating erythrocytes (Harlow & Lane, Antibodies: A Laboratory Manual, CSHL Press, 1988; www.who.int/csr/resources/publications/influenza/WHO_CDS_CSR_NCS_2002_5/en/in dex.html; www.who.int/csr/disease/avian_influenza/guidelines/labtests/en/index.html). In other embodiments, such agents may include nucleic acids that specifically bind to nucleotides that encode particular HA polypeptides and that can be used to specifically detect such HA polypeptides by RT-PCR or *in situ* hybridization (www.who.int/csr/resources/publications/influenza/WHO_CDS_CSR_NCS_2002_5/en/i ndex.html; www.who.int/csr/disease/avian_influenza/guidelines/labtests/en/index.html). In certain embodiments, nucleic acids which have been isolated from a sample are amplified prior to detection. In certain embodiments, diagnostic reagents can be detectably labeled.

The present disclosure also provides kits containing reagents according to the present disclosure for the generation of influenza viruses and vaccines. Contents of such kits include, but are not limited to, expression plasmids containing HA encoding HA polypeptides of interest (nucleotides (or fragments, such as characteristic fragments). Alternatively or additionally, kits may contain expression plasmids that express HA polypeptides of interest (or characteristic or biologically active portions). Expression plasmids containing no virus genes may also be included so that users are capable of incorporating HA nucleotides from any influenza virus of interest. Mammalian cell lines may also be included with the kits, including but not limited to, Vero and MDCK cell lines. In certain embodiments, diagnostic reagents can be detectably labeled.

In certain embodiments, kits for use in accordance with the present invention may include, a reference sample, instructions for processing samples, performing the test, instructions for interpreting the results, buffers and/or other reagents necessary for performing the test. In certain embodiments the kit can comprise a panel of antibodies.

In some embodiments of the present invention, glycan arrays, as discussed above, may be utilized as diagnostics and/or kits.

In certain embodiments, inventive glycan arrays and/or kits are used to perform dose response studies to assess binding of HA polypeptides to umbrella glycans at multiple doses (e.g., as described herein). Such studies give particularly valuable insight into the binding characteristics of tested HA polypeptides, and are particularly useful to assess specific binding. Dose response binding studies of this type find many useful applications. To give but one example, they can be helpful in tracking the evolution of binding characteristics in a related series of HA polypeptide variants, whether the series is generated through natural evolution, intentional engineering, or a combination of the two.

In certain embodiments, inventive glycan arrays and/or kits are used to induce, identify, and/or select binding agents (*e.g.,* HA polypeptides, and/or HA polypeptide variants) having desired binding characteristics. For instance, in some embodiments, inventive glycan arrays and/or kits are used to exert evolutionary (e.g., screening and/or selection) pressure on a population of polypeptide binding agents (*e.g.,* HA polypeptides).

In some embodiments, provided kits comprise instructions for use.

Therapeutic Compositions

The present disclosure provides a variety of compositions that comprise or otherwise deliver HA polypeptides or fragments thereof, detecting agents, competing agents, etc as described herein. In some embodiments, provided compositions are useful in the treatment of influenza infection, prior to or after initiation of infection and/or development of one or more symptoms of infection.

The invention encompasses treatment of influenza infections by administration of such inventive therapeutic compositions. In some embodiments, inventive therapeutic compositions are administered to a subject suffering from or susceptible to an influenza infection. In some embodiments, a subject is considered to be suffering from an influenza infection in the subject is displaying one or more symptoms commonly associated with influenza infection. In some embodiments, the subject is known or believed to have been exposed to the influenza virus. In some embodiments, a subject is considered to be susceptible to an influenza infection if the subject is known or believed to have been exposed to the influenza virus. In some embodiments, a subject is known or believed to have been exposed to the influenza virus if the subject has been in contact with other individuals known or suspected to have been infected with the influenza virus and/or if the subject is or has been present in a location in which influzena infection is known or thought to be prevalent.

In some embodiments, subjects suffering from or susceptible to influenza infection are tested for antibodies to inventive binding agents prior to, during, or after administration of inventive therapeutic compositions. In some embodiments, subjects having such antibodies are not administered therapeutic compositions comprising inventive binding agents. In some embodiments, an appropriate dose of pharmaceutical composition and/or binding agent is selected based on detection (or lack thereof) of such antibodies.

In some embodiments, selection of a particular subject for treatment, particular binding agent or composition for administration, and/or particular dose or regimen for administration, is memorialized, for example in a written, printed, or electronic storage form.

Inventive therapeutic compositions may be admininstered prior to or after development of one or more symptoms of influenza infection.

In general, a therapeutic composition will include a therapeutic agent in addition to one or more inactive agents such as a sterile, biocompatible carrier including, but not limited to, sterile water, saline, buffered saline, or dextrose solution. Alternatively or additionally, the composition can contain any of a variety of additives, such as stabilizers, buffers, excipients (e.g., sugars, amino acids, etc), or preservatives.

Exemplary inactive agents include, for example, a sterile, biocompatible carrier including, but not limited to, sterile water, saline, buffered saline, or dextrose solution. Alternatively or additionally, any of a variety of solvents, dispersion media, diluents, or other liquid vehicles, dispersion or suspension aids, disintegrating agents, surface active agents, isotonic agents, thickening or emulsifying agents, preservatives, buffering agents, solid binders, granulating agents, lubricants, coloring agents, sweetening agents, flavoring agents, perfuming agents, and the like, may be utilized, as suited to the particular formulation or dosage form desired. Remington's The Science and Practice of Pharmacy, 21st Edition, A. R. Gennaro, (Lippincott, Williams & Wilkins, Baltimore, MD, 2006) discloses various excipients used in formulating therapeutic compositions and known techniques for the preparation thereof. Except insofar as any conventional excipient medium is incompatible with a substance or its derivatives, such as by producing any undesirable biological effect or otherwise interacting in a deleterious manner with any other component of the pharmaceutical composition, its use is contemplated to be within the scope of this invention.

In some embodiments, a pharmaceutical composition will include a therapeutic agent that is encapsulated, trapped, or bound within a lipid vesicle, a bioavailable and/or biocompatible and/or biodegradable matrix, or other microparticle.

In some embodiments, provided compositions further comprise one or more adjuvants. Any adjuvant may be used in accordance with the present invention. A large number of adjuvants are known; a useful compendium of many such compounds is prepared by the National Institutes of Health and can be found on the internet (www.niaid.nih.gov/daids/vaccine/pdf/compendium.pdf). See also Allison (1998, Dev. Biol. Stand., 92:3-11), Unkeless et al. (1998, Annu. Rev. Immunol., 6:251-281), and Phillips et al. (1992, Vaccine, 10:151-158). Hundreds of different adjuvants are known in the art and could be employed in the practice of the present invention.

Therapeutic compositions may be administered using any amount and any route of administration effective for treatment and/or vaccination. The exact amount required will vary from subject to subject, depending on the species, age, and general condition of the subject, the severity of the infection, the particular composition, its mode of administration, its mode of activity, and the like. Therapeutic compositions are typically formulated in dosage unit form for ease of administration and uniformity of dosage. It will be understood, however, that the total daily usage of the compositions of the present disclosure will be decided by the attending physician within the scope of sound medical judgment. The specific therapeutically effective dose level for any particular subject or organism will depend upon a variety of factors including the disorder being treated and/or vaccinated and the severity of the disorder; the activity of the specific vaccine composition employed; the half-life of the composition after administration; the age, body weight, general health, sex, and diet of the subject; the time of administration, route of administration, and rate of excretion of the specific compound employed; the duration of the treatment; drugs used in combination or coincidental with the specific compound employed; and like factors, well known in the medical arts.

Therapeutic compositions of the present disclosure may be administered by any route. In some embodiments, therapeutic compositions of the present disclosure are administered by a variety of routes, including oral (PO), intravenous (IV), intramuscular (IM), intra-arterial, intramedullary, intrathecal, subcutaneous (SQ), intraventricular, transdermal, interdermal, intradermal, rectal (PR), vaginal, intraperitoneal (IP), intragastric (IG), topical (*e.g.,* by powders, ointments, creams, gels, lotions, and/or drops), mucosal, intranasal, buccal, enteral, vitreal, sublingual; by intratracheal instillation, bronchial instillation, and/or inhalation; as an oral spray, nasal spray, and/or aerosol, and/or through a portal vein catheter.

At present the oral or nasal spray or aerosol route (e.g., by inhalation) is most commonly used to deliver therapeutic agents directly to the lungs and respiratory system. However, the present disclosure encompasses the delivery of the inventive pharmaceutical composition by any appropriate route taking into consideration likely advances in the sciences of drug delivery.

In some embodiments, preparations for inhaled or aerosol delivery comprise a plurality of particles. In some embodiments, such preparations have a mean particle size of 4, 5, 6, 7, 8, 9, 10, 11, 12, or 13 microns. In some embodiments, preparations for inhaled or aerosol delivery are formulated as a dry powder. In some embodiments, preparations for inhaled or aerosol delivery are formulated as a wet powder, for example through inclusion of a wetting agent. in some embodiments, the wetting agent is selected from the group consisting of water, saline, or other liquid of physiological pH.

In some embodiments, inventive compositions are administered as drops to the nasal or buccal cavity. In some embodiments, a dose may comprise a plurality of drops (e.g., 1-100, 1-50, 1-20, 1-10, 1-5, etc.)

In some embodiments, inventive compositions are administered using a device that delivers a metered dosage of composition (e.g., of binding agent).

Suitable devices for use in delivering intradermal therapeutic compositions described herein include short needle devices such as those described in U.S. Pat. No. 4,886,499, U.S. Pat. No. 5,190,521, U.S. Pat. No. 5,328,483, U.S. Pat. No. 5,527,288, U.S. Pat. No. 4,270,537, U.S. Pat. No. 5,015,235, U.S. Pat. No. 5,141,496, U.S. Pat. No. 5,417,662. Intradermal compositions may also be administered by devices which limit the effective penetration length of a needle into the skin, such as those described in WO99/34850, and functional equivalents thereof. Also suitable are jet injection devices which deliver liquid vaccines to the dermis via a liquid jet injector or via a needle which pierces the stratum corneum and produces a jet which reaches the dermis. Jet injection devices are described for example in U.S. Pat. No. 5,480,381, U.S. Pat. No. 5,599,302, U.S. Pat. No. 5,334,144, U.S. Pat. No. 5,993,412, U.S. Pat. No. 5,649,912, U.S. Pat. No. 5,569,189, U.S. Pat. No. 5,704,911, U.S. Pat. No. 5,383,851, U.S. Pat. No. 5,893,397, U.S. Pat. No. 5,466,220, U.S. Pat. No. 5,339,163, U.S. Pat. No. 5,312,335, U.S. Pat. No. 5,503,627, U.S. Pat. No. 5,064,413, U.S. Pat. No. 5,520,639, U.S. Pat. No. 4,596,556, U.S. Pat. No. 4,790,824, U.S. Pat. No. 4,941,880, U.S. Pat. No. 4,940,460, WO 97/37705 and WO 97/13537. Also suitable are ballistic powder/particle delivery devices which use compressed gas to accelerate vaccine in powder form through the outer layers of the skin to the dermis. Additionally, conventional syringes may be used in the classical mantoux method of intradermal administration.

General considerations in the formulation and manufacture of pharmaceutical agents may be found, for example, in Remington's Pharmaceutical Sciences, 19th ed., Mack Publishing Co., Easton, PA, 1995.

Inventive therapeutic compositions may be administered in any dose appropriate to achieve a desired outcome. In some embodiments, the desired outcome is reduction in intensity, severity, and/or frequency, and/or delay of onset of one or more symptoms of infection (e.g., influenza infection).

Therapeutic compositions in accordance with the present disclosure may be administered either alone or in combination with one or more other therapeutic agents. By "in combination with," it is not intended to imply that the agents must be administered at the same time and/or formulated for delivery together, although these methods of delivery are within the scope of the disclosure. Compositions can be administered concurrently with, prior to, or subsequent to, one or more other desired therapeutics or medical procedures. In will be appreciated that therapeutically active agents utilized in combination may be administered together in a single composition or administered separately in different compositions. In general, each agent will be administered at a dose and/or on a time schedule determined for that agent.

In some embodiments, inventive therapeutic compositions are formulated to reduce immunogenicity of included agents. For example, in some embodiments, an included active agent is associated with (e.g., bound to) an agent, such as polyethylene glycol and/or carboxymethyl cellulose, that masks its immunogenicity. In some embodiments, an included active agent has additional glycosylation that reduces immunogenicity.

In some embodiments, the present disclosure provides kits for administration of inventive therapeutic compositions. For example, in some embodiments, the present disclosure provides a kit comprising at least one dose of a binding agent. In some embodiments, the present disclosure provides a kit comprising an initial unit dose and a subsequent unit dose of a binding agent. In some such embodiments, the initial unit dose is greater than the subsequent unit dose or wherein the two doses are equal.

In some embodiments, inventive kits (particularly those for administration of inventive compositions) comprise at least one component of a delivery device, e.g., an inhaler. In some such embodiments, the present disclosure provides a kit comprising at least one component of a delivery device, e.g., an inhaler and a dose of an of a binding agent.

Vaccine Compositions

In some particular embodiments, the present invention provides therapeutic compositions that are vaccine compositions for use in preventing influenza infections, particularly when such infections are mediated by a human-adapted H5 HA. In some embodiments, such a vaccine composition comprises one or more provided HA polypeptides or fragments thereof, nucleic acids that encode them, expression systems that produce them, and/or competing agents that compete their interactions with one or more HA receptors. In some embodiments, such a vaccine composition comprises one or more adjuvants for promoting or stimulating an immune response in an individual to whom the vaccine composition is administered.

In some embodiments, the present invention provides for vaccines and the administration of these vaccines to a human subject. In certain embodiments, vaccines are compositions comprising one or more of the following: (1) inactivated virus, (2) live attenuated influenza virus, for example, replication-defective virus, (3) inventive HA polypeptides or fragments thereof, detecting agents, binding agents, nucleic acids, expression systems, cells or organisms as described herein.

In some embodiments, the present invention provides inactivated flu vaccines. In certain embodiments, inactivated flu vaccines comprise one of three types of antigen preparation: inactivated whole virus, sub-virions where purified virus particles are disrupted with detergents or other reagents to solubilize the lipid envelope ("split" vaccine) or purified HA polypeptide ("subunit" vaccine). In certain embodiments, virus can be inactivated by treatment with formaldehyde, beta-propiolactone, ether, ether with detergent (such as Tween-80), cetyl trimethyl ammonium bromide (CTAB) and Triton N101, sodium deoxycholate and tri(n-butyl) phosphate. Inactivation can occur after or prior to clarification of allantoic fluid (from virus produced in eggs); the virions are isolated and purified by centrifugation (Nicholson et al., eds., Textbook of Influenza, Blackwell Science, Malden, MA, 1998). To assess the potency of the vaccine, the single radial immunodiffusion (SRD) test can be used (Schild et al., Bull. World Health Organ., 52:43-50 & 223-31, 1975; Mostow et al., J. Clin. Microbiol., 2:531, 1975).

The present invention also provides live, attenuated flu vaccines, and methods for attenuation are well known in the art. In certain embodiments, attenuation is achieved through the use of reverse genetics, such as site-directed mutagenesis.

In some embodiments, influenza virus for use in vaccines is grown in eggs, for example, in embryonated hen eggs, in which case the harvested material is allantoic fluid. Alternatively or additionally, influenza virus may be derived from any method using tissue culture to grow the virus. Suitable cell substrates for growing the virus include, for example, dog kidney cells such as MDCK or cells from a clone of MDCK, MDCK-like cells, monkey kidney cells such as AGMK cells including Vero cells, cultured epithelial cells as continuous cell lines, 293T cells, BK-21 cells, CV-1 cells, or any other mammalian cell type suitable for the production of influenza virus (including upper airway epithelial cells) for vaccine purposes, readily available from commercial sources (e.g., ATCC, Rockville, Md.). Suitable cell substrates also include human cells such as MRC-5 cells. Suitable cell substrates are not limited to cell lines; for example primary cells such as chicken embryo fibroblasts are also included.

In some embodiments, inventive vaccines further comprise one or more adjuvants. For example, aluminum salts (Baylor et al., Vaccine, 20:S18, 2002) and monophosphoryl lipid A (MPL; Ribi et al., (1986, Immunology and Immunopharmacology of bacterial endotoxins, Plenum Publ. Corp., NY, p407, 1986) can be used as adjuvants in human vaccines. Alternatively or additionally, new compounds are currently being tested as adjuvants in human vaccines, such as MF59 (Chiron Corp., http://www.chiron.com/investors/pressreleases/2005/051028.html), CPG 7909 (Cooper et al., Vaccine, 22:3136, 2004), and saponins, such as QS21 (Ghochikyan et al., Vaccine, 24:2275, 2006).

Additionally, some adjuvants are known in the art to enhance the immunogenicity of influenza vaccines, such as poly[di(carboxylatophenoxy)phosphazene] (PCCP; Payne et al., Vaccine, 16:92, 1998), interferon-$\gamma$ (Cao et al., Vaccine, 10:238, 1992), block copolymer P1205 (CRL1005; Katz et al., Vaccine,. 18:2177, 2000), interleukin-2 (IL-2; Mbwuike et al., Vaccine, 8:347, 1990), and polymethyl methacrylate (PMMA; Kreuter et al., J. Pharm. Sci., 70:367, 1981).

In some embodiments, inventive vaccine compositions do not include adjuvants (e.g., provided compositions are essentially free of adjuvants). In some embodiments, inventive vaccine compositions do not include an alum adjuvant (e.g., provided compositions are essentially free of alum).

In some embodiments, vaccine compositions are formulated or otherwise designed or prepared for administration prior to symptoms, and/or to exposure. It will be appreciated by those skilled in the art, however, that in many embodiments vaccine compositions may alternatively or additionally be administered after exposure, infection, and/r development of symptoms.

*Combination Therapy*

Therapeutic compositions as described herein may be administered either alone or in combination with one or more other therapeutic agents including, but not limited to, vaccines and/or antibodies. By "in combination with," it is not intended to imply that the agents must be administered at the same time or formulated for delivery together, although these methods of delivery are within the scope of the invention. In general, each agent will be administered at a dose and on a time schedule determined for that agent. Additionally, the invention encompasses the delivery of inventive therapeutic compositions in combination with agents that may improve their bioavailability, reduce or modify their metabolism, inhibit their excretion, or modify their distribution within the body. Although the therapeutic compositions of the present disclosure can be used for treatment of any subject (e.g., any animal) in need thereof, they are most preferably used in the treatment of humans. In some embodiments, inventive therapeutic compositions are administered in combination with one or more of an anti-viral agent (e.g., Oseltamivir [tamiflu], Zanamavir [Releza], etc.) and/or a sialydase. In some embodiments, inventive therapeutic compositions are administered in a combination with one or more other therapies (e.g., pain relievers, decongenstants, cough suppressants, sleep aids, etc) commonly used to treat influenza infection or symptoms thereof.

*Uses*

In some embodiments, the present disclosure provides technologies and methodologies for treating, monitoring and even predicting evolution of sequences of avian influenza HA strains.

*Treatment of Influenza Infections*

The present disclosure provides methods of treating influenza infection. In certain embodiments, such methods involve administering one or more inventive HA polypeptides or fragments thereof, nucleic acids that encode them, expression systems that produce them, and/or competing agents that compete their interactions with one or more HA receptors to a subject in need thereof. In some embodiments, HA polypeptides or fragments thereof, nucleic acids that encode them, expression systems that produce them, and/or competing agents that compete their interactions with one or more HA receptors inhibit the ability of HA (e.g. HA expressed on the surface of influenza virus) to bind to umbrella-topology glycans (e.g. glycans associated with human upper respiratory epithelial tissues, such as trachea and bronchus).

In some embodiments, HA polypeptides or fragments thereof, nucleic acids that encode them, expression systems that produce them, and/or competing agents that compete their interactions with one or more HA receptors of the present disclosure are used in the treatment of one or more of the following symptoms: fever, sore throat, muscle pains, severe headache, coughing, weakness, general discomfort, pneumonia, nausea, and/or vomiting. In certain embodiments, these symptoms are caused by influenza infection.

In some embodiments, inventive pharmaceutical compositions are administered to a subject suffering from or susceptible to an influenza infection. In some embodiments, a subject is considered to be suffering from an influenza infection in the subject is displaying one or more symptoms commonly associated with influenza infection. In some embodiments, the subject is known or believed to have been exposed to the influenza virus. In some embodiments, a subject is considered to be susceptible to an influenza infection if the subject is known or believed to have been exposed to the influenza virus. In some embodiments, a subject is known or believed to have been exposed to the influenza virus if the subject has been in contact with other individuals known or suspected to have been infected with the influenza virus and/or if the subject is or has been present in a location in which influenza infection is known or thought to be prevalent.

In some embodiments, the present disclosure provides a method of treating influenza infection comprising steps of (1) providing a patient exhibiting symptoms of influenza infection, and (2) administering a therapeutic amount of one or more HA polypeptides or fragments thereof, nucleic acids that encode them, expression systems that produce them, and/or competing agents that compete their interactions with one or more HA receptors to the patient. In some embodiments, the present disclosure provides a method of treating influenza infection comprising steps of (1) providing a patient suffering from influenza infection, and (2) administering a therapeutic amount of one or more HA polypeptides or fragments thereof, nucleic acids that encode them, expression systems that produce them, and/or competing agents that compete their interactions with one or more HA receptors to the patient. In some embodiments, the present disclosure provides a method of treating influenza infection comprising steps of (1) providing a patient susceptible to influenza infection, and (2) administering a therapeutic amount of one or more HA polypeptides or fragments thereof, nucleic acids that encode them, expression systems that produce them, and/or competing agents that compete their interactions with one or more HA receptors to the patient.

In some embodiments, the present disclosure provides methods of treating influenza infection comprising steps of (1) providing a patient exhibiting symptoms of, suffering from, and/or susceptible to influenza infection, and (2) administering a substance that competes away the binding of HA polypeptides (e.g. HA polypeptides associated with influenza virus particles) with umbrella-topology glycans in human upper respiratory tissues.

In some embodiments, the present disclosure provides a method of preventing and/or delaying the onset of influenza infection comprising steps of (1) providing a patient susceptible to influenza infection, and (2) administering a therapeutic amount of one or more HA polypeptides or fragments thereof, nucleic acids that encode them, expression systems that produce them, and/or competing agents that compete their interactions with one or more HA receptors to the patient.

*Surveillance/Monitoring*

Prior to the present invention, the effects of mutations on the quantitative glycan-receptor binding affinity of H5 HA's had not been characterized. Presented herein, among other things, are methods for defining and understanding the requirements for an H5 HA to quantitatively switch its binding preference to human receptors in a manner characteristic of human adapted HAs in the context of the changes in the molecular environment of the receptor binding site. In some embodiments, a combination of structural and inter-residue interaction network analyses are combined to define mutations in the receptor binding site of H5 HA that can quantitatively switch its glycan receptor binding preference to human receptors in a manner similar to pandemic H1 and H2 HA strains.

In some embodiments, the present invention provides methods of monitoring a population for human infective and/or human transmissible influenza. In some embodiments, methods of determining pandemic risk from a strain of influenza are provided. In some embodiments, a method of monitoring influenza includes the steps of obtaining a sample from a source suspected to contain influenza, contacting the sample with one or more agents that specifically binds to an H5 HA polypeptide, detecting the binding of the agent with the sample, so that the presence and/or level of H5 HA in the sample is determined. In some embodiments, binding of the one or more agents to the sample indicates the presence of a human infective H5 HA. In some embodiments, binding of the one or more agents to the sample indicates the absence of a human infective H5 HA.

In some embodiment, methods according to the present invention may be used to analyze any of a variety of sample sources including environmental sources, human patient sources, or animal sources, for example. In some embodiments, analysis of one or more samples occurs at least twice. In some embodiments, each analysis is separated by a period of time to allow for longitudinal monitoring of a subject or population, for example. In some embodiments, the period of time may be: 1 hour, 12 hours, 1 day, 2 days, 3 days, 4 days, 5 days, 6 days, 1 week, 2 weeks, 3 weeks, 1 month, 2 months, 3 months, 4 months, 5 months, 6 months, or 1 year.

*Detecting and/or Characterizing Useful Agents and/or Interactions*

The present disclosure provides a variety of technologies for identification and/or characterization of useful agents (e.g., agents useful in the treatment, prevention, and/or analysis of influenza infection) and/or interactions.

For example, a variety of binding studies and/or formats are useful for the identification and/or characterization of useful agents as described herein. In some embodiments, the present disclosure utilizes systems for analyzing binding interactions between HA polypeptides and HA receptors. In some such embodiments, analysis methods comprise steps of 1) providing a source of HA polypeptides or binding components thereof; 2) providing a source of HA receptors or binding components thereof; and 3) contacting the provided sources with one another under conditions and for a time sufficient that binding between the HA polypeptides (or binding components thereof) and HA receptors (or binding components thereof) can be assessed. Such approaches can be utilized, for example, to identify or characterize HA polypeptides, in particular variant HA polypeptides, of interest, and/or to identify and/or characterize agents that bind thereto and/or inhibit interaction thereof with HA receptors.

In some embodiments, suitable sources of HA polypeptides or binding components thereof include, but are not limited to, pathological samples, such as blood, serum/plasma, peripheral blood mononuclear cells/peripheral blood lymphocytes (PBMC/PBL), sputum, urine, feces, throat swabs, dermal lesion swabs, cerebrospinal fluids, cervical smears, pus samples, food matrices, and tissues from various parts of the body such as brain, spleen, and liver. Alternatively or additionally, other suitable sources for samples containing HA polypeptides include, but are not limited to, environmental samples such as soil, water, and flora. Yet other samples include laboratory samples, for example of engineered HA polypeptides designed and/or prepared by researchers. Other samples that have not been listed may also be applicable. In some embodiments, sources (and/or samples contacted with HA receptors or binding components thereof) comprise intact virus or virus-like particles; in some embodiments, such sources and/or samples comprises HA polypeptides. In some embodiments, HA polypeptides are utilized in trimer form.

In some embodiments, suitable sources of HA receptors or binding components thereof include tissue samples; in some embodiments, suitable sources include isolated HA receptors or binding components thereof. In some embodiments, suitable sources include collections of glycans, for example in glycan arrays, comprising HA receptor glycans. In some embodiments, suitable sources include glycan collections comprising $\alpha$2-3-linked and/or $\alpha$2-6-linked glycans. In some embodiments, suitable sources include glycan collections comprising cone-topology and/or umbrella-topology glycans. In some embodiments, suitable sources include glycans found on human upper respiratory tract HA receptors.

It will be appreciated that a variety of binding interactions can usefully be studied in accordance with the present invention. In addition to HA polypeptide-HA receptor interactions, various antibody-antigen interaction or other ligand-target interactions may be studied, as described herein. For example, interactions between HA polypeptides and detecting or competing agents may be analyzed, in the presence or absence of HA receptors (or binding components thereof)

In some embodiments, one or both interacting components utilized in a binding study is detectably labeled (directly or indirectly) prior to, during, or after the contacting step. In some such embodiments, at least one interacting component is spatially localized, for example on an array. To give but one example, in some embodiments, a detectably labeled HA polypeptide or binding component thereof is contacted with a collection of glycans, for example on an array in which different glycans are distinctly localized. In some such embodiments, binding can be assessed by detecting and/or quantifying localized label (e.g., using a scanning device).

Alternatively or additionally, binding between or among interacting components or entities can be measured using, for example, calorimetric, fluorescence, or radioactive detection systems, or other labeling methods, or other methods that do not require labeling. In general, fluorescent detection typically involves utilizing a first interacting partner (e.g., an HA polypeptide or binding component thereof, or an HA receptor or binding portion thereof) that is or becomes labeled with a fluorescent molecule and monitoring fluorescent signals. Alternatively or additionally, one or both of the interacting components or entities can be tagged with a tag (e.g., biotin or streptavidin, antigen epitope, nucleic acid, etc) that itself interacts detectably with a partner (e.g., streptaviding or biotin, andibody, completmentary nucleic acid).

In some embodiments, fluorescence quenching methods can be utilized in which one interacting component or entity is fluorescently labeled and the other is provided in a context that squelches the fluorescence if/when binding occurs.

Alternatively or additionally, binding studies can utilize live cells or tissue samples that have been grown in the presence of a radioactive substance, yielding a radioactively labeled probe. Binding in such embodiments can be detected by measuring radioactive emission.

Such methods are useful to determine the fact of binding and/or the extent of binding between interacting components or entities. In some embodiments, such methods can further be used to identify and/or characterize agents that interfere with or otherwise alter interactions of interest.

Methods described herein may be of particular use in, for example, identifying whether a molecule thought to be capable of interacting with a carbohydrate can actually do so, or to identify whether a molecule unexpectedly has the capability of interacting with a carbohydrate.

The present disclosure also provides methods of using glycan collections, for example, to detect a particular agent in a test sample. For instance, such methods may comprise steps of (1) contacting a collection of glycans (e.g., a glycan array) with a test sample (e.g., with a sample known or thought to contain an HA polypeptide); and, (2) detecting the binding of any agent in the test sample to the glycan collection.

Binding studies may be utilized in accordance with the present disclosure, for example, to determine kinetics of interaction between binding agent and glycan. For example, inventive methods for determining interaction kinetics may include steps of (1) contacting a glycan collection with a sample comprising the agent being tested; and, (2) measuring kinetics of interaction between the binding agent and the glycan(s).

The kinetics of interaction of between binding entities or components (e.g., a binding agent and glycans in a collection, for example on an array) can be measured by real time changes in, for example, colorimetric or fluorescent signals, as detailed above. Such methods may be of particular use in, for example, determining whether a particular binding agent is able to interact with a specific carbohydrate with a higher degree of binding than does a different binding agent interacting with the same carbohydrate.

In some embodiments, binding studies as described herein, and particularly binding studies that characterize interactions between HA polypeptides or binding components thereof and HA receptors or binding components thereof, are performed over a range of concentrations of one or both binding components or entities.

In some embodiments, binding (and/or infection and/or transmission) studies are performed in or utilizing an animal hose. As used herein, an "animal host" includes any animal model suitable for influenza research. For example, animal hosts suitable for the disclosure can be any mammalian hosts, including primates, ferrets, cats, dogs, cows, horses, rodents such as, mice, hamsters, rabbits, and rats. In certain embodiments, an animal host used for the disclosure is a ferret. In particular, in some embodiments, an animal host is naive to viral exposure or infection prior to administration of an inventive binding agent (optionally in an inventive composition). In some embodiments, the animal host is inoculated with, infected with, or otherwise exposed to virus prior to or concurrent with administration of an inventive binding agent. An animal host used in the practice of the present disclosure can be innoculated with, infected with, or otherwise exposed to virus by any method known in the art. In some embodiments, an animal host may be innoculated with, infected with, or exposed to virus intranasally.

In some embodiments, a suitable animal host may have a similar distribution of umbrella vs. cone topology glycans and/or $\alpha$2-6 glycans vs. $\alpha$ 2-3 glycans to the distribution found in the human respiratory tract. For example, it is contemplated that a ferret as an animal host may be more representative than a mouse when used as model of disease caused by influenza viruses in humans (Tumpey, et al. Science (2007) 315; 655-659). Without wishing to be bound any theories, the present disclosure encompasses the idea that ferrets may have a more similar distributution of glycans in the respiratory tract to those in the human respiratory tract than mouse does to human.

Naive and/or innoculated animals may be used for any of a variety of studies. For example, such animal models may be used for virus transmission studies as in known in the art. It is contemplated that the use of ferrets in virus transmission studies may serve as a reliable predictor for virus transmission in humans. For example, air transmission of viral influenza from innoculated animals (e.g., ferrets) to naive animals is known in the art (Tumpey, et al. Science (2007) 315; 655-659). Virus transmission studies may be used to test inventive binding agent poylpeptides (e.g., HA polypeptides). For example, inventive binding agents may be administered to a suitable animal host before, during or after virus transmission studies in order to determine the efficacy of said binding agent in blocking virus binding and/or infectivity in the animal host. Using information gathered from virus transmission studies in an animal host, one may predict the efficacy of a binding agent in blocking virus binding and/or infectivity in a human host.

*Production of polypeptides*

Provided polypeptides (e.g., HA polypeptides, antibodies, etc, and/or fragments, such as characteristic fragments, thereof) thereof, may be produced by any available means.

Polypeptides may be produced, for example, by utilizing a host cell system engineered to express nucleic acids encoding a polypeptide of interest. In some embodiments, such encoding nucleic acids are heterologous to the host cell system and are introduced into the system through action of the hand of man. Alternatively or additionally, the host cell system may be manipulated to express the encoding polypeptide at a particular (e.g., elevated) level and/or at a particular time.

Those skilled in the art will be aware of a wide variety of host cell systems that can appropriately be used to produce polypeptides as described herein. For example, polypeptides may be produced in microbial, mammalian, avian, or plant cell systems. In some embodiments, eukaryotic cell systems are utilized. In some embodiments, utilized cell systems are or comprise intact tissues and/or organisms. To give but a few examples, in some embodiments, provided polypeptides are expressed in egg, baculovirus, plant, yeast, Madin-Darby Canine Kidney cells (MDCK), or Vero (African green monkey kidney) cell systems.

Alternatively or additionally, provided polypeptides may be synthesized *in vitro,* for example utilizing *in vitro* transcription and/or translation systems and/or through chemical synthesis.

In some embodiments, provided HA polypeptides (or certain fragments thereof) may be produced in the context of intact virus or virus-like particles.

In some embodiments, provided HA polypeptides (or certain fragments thereof) can be isolated and/or purified from influenza virus. For example, virus may be grown in eggs, such as embryonated hen eggs, in which case the harvested material is typically allantoic fluid. Alternatively or additionally, virus may be grown in a tissue culture system. Suitable cell substrates for growing the virus include, for example, dog kidney cells such as MDCK or cells from a clone of MDCK, MDCK-like cells, monkey kidney cells such as AGMK cells including Vero cells, cultured epithelial cells as continuous cell lines, 293T cells, BK-21 cells, CV-1 cells, or any other mammalian cell type suitable for the production of influenza virus for vaccine purposes, readily available from commercial sources (e.g., ATCC, Rockville, Md.). Suitable cell substrates also include human cells such as MRC-5 cells. Suitable cell substrates are not limited to cell lines; for example primary cells such as chicken embryo fibroblasts are also included.

Also, it will be appreciated by those of ordinary skill in the art that polypeptides, and particularly HA polypeptides as described herein, may be generated, identified, isolated, and/or produced by culturing cells or organisms that produce the polypeptide (whether alone or as part of a complex, including as part of a virus particle or virus), under conditions that allow ready screening and/or selection of polypeptides that show desired binding and/or activity characteristics as described herein.

**Exemplification**

*Example 1: Design and Characterization of Novel H5 HA Variants*

Introduction

Influenza A virus poses a significant threat to global health both from the standpoint of seasonal outbreaks and also from that of the ability of avian viruses (antigenically novel to humans) to adapt to the human host. The sudden emergence of the antigenically novel 2009 H1N1 strain from multiple reassortment of influenza gene segments from avian, swine, and human viruses substantially impacted the global economy and highlighted the critical need for proper surveillance to be more prepared for such spontaneous pandemic outbreaks in the future. A characteristic property of human-adapted viruses such as H1N1, H2N2, and H3N2 is the quantitatively high binding affinity of the viral HA to human receptors in relation to its low to minimal binding to avian receptors.

Amino acid mutations in viral surface glycoprotein (HA) and polymerase (PB2) were able to confer aerosol transmission to avian H1N1 influenza isolate (Van Hoeven et al., 2009 PNAS, 106:3366). Among the avian subtypes known to infect humans, H5N1 has the highest mortality rate. It is therefore vital to implement new strategies for improved monitoring of the evolution of the H5N1 viruses and to track its potential to adapt to the human host.

Highly pathogenic H5N1 influenza A virus subtype poses a global health concern given that it has already led to several localized outbreaks in humans with a high mortality rate (~60%) since 2003 (Neumann et al., 2010 Cell Res., 20:51; Guan et al., 2009 Rev Sci Tech, 28:39). However the H5N1 subtype has not yet adapted to the human host so as to establish sustained human-to-human transmission via respiratory droplets (or aerosol transmission).

The HA from avian subtypes typically binds to $\alpha2\rightarrow3$ sialylated glycans (or *avian receptors*)(Ge et al., 2011 Crit Rev Microbiol., 37:157). A hallmark feature of human-adapted subtypes such as H1N1, H2N2, and H3N2 is the quantitative switch in their binding preference to $\alpha2\rightarrow6$ sialylated glycan receptors (or *human receptors*) which is defined by high relative binding affinity to human receptors over avian receptors. This quantitative switch has been shown to correlate with respiratory droplet transmissibility of the pandemic H1N1 and H2N2 viruses in ferrets(Tumpey et al., 2007 Science, 315:655; Srinivasan et al., 2008 PNAS, 105:2800; Pappas et al., 2010 PLoS One, 5:e11158; Viswanathan et al., 2010 PLoS One, 5:e13768). Therefore, a necessary determinant of human adaptation of avian-adapted H5N1 subtypes is for its HA to acquire mutations which quantitatively switch its binding preference to human receptors (Ge et al., 2011 Crit Rev Microbiol., 37:157; Shriver et al., 2009 Chem Biol., 16:803). Identifying mutations that switch glycan receptor-binding specificity of H5 HA has been the focus of several previous studies (Gambaryan et al., 2006 Virology, 344:432; Stevens et al., 2006 Science, 312:404; Yamada et al., 2006 Nature, 444:378; Chandrasekaran et al., 2008 Nat Biotechnol., 26:107; Stevens et al., 2008 J Mol. Biol., 381:1382; Wang et al., 2009 PNAS, 106:18137; Watanabe et al., 2012 Trends In Microbiology, 20:11). Some of these studies include analyses of glycan receptor binding of H5 HAs with natural variations in the receptor-binding site (RBS) (Yamada et al., 2006 Nature, 444:378). Other studies have mutated H5 HA to include either the hallmark changes for human adaptation of H2/H3 HA (Q226L and G228S or *LS*) and/or H1 HA (E190D, G225D or *DD*). None of the variant H5 HAs, whether engineered or found in a natural source) have shown a quantitative switch in binding to human receptors in a fashion characteristic of human-adapted 'pandemic' strain, HAs (such as 1918 H1N1, 1958 H2N2, and 2009 H1N1, see **FIG. 1**).

Recently, two studies by Imai *et al.* (Imai et al., 2012 Nature, In Press) and Herfst *et al.* (Herfst et al., 2012 Science, 336:1534) demonstrated that specific sets of mutations in HA from human H5N1 isolates A/Vietnam/1203/2004 (Viet04) and A/Indonesia/5/2005 (Ind05) respectively switch receptor preference and confer respiratory droplet viral transmission in ferrets to the viruses possessing these variant H5 HAs. It is evident from these studies that differences in genetic background and selection pressure strategies give rise to distinct sets of amino acid changes in Viet04 and Ind05 HAs that are associated with aerosol transmission in ferrets. Based on these studies, another study by Russell et al. (Russell et al., 2012 Science, 336:1541) used mathematical models and statistical analysis of HA and polymerase PB2 nucleotide sequences to evaluate the potential for H5N1 strains, in the context of their phylogenetic divergence, to acquire the specific amino acid changes reported for Viet04 and Ind05. Based on the sequence analyses, this study points out that there is a clear sequence divergence in current H5N1 HAs (such as clade 2.2.1) and that the genetic changes identified in Viet04 and Ind05 HA may not be the only ones that lead to respiratory droplet transmission.

The evolution of H5 HA sequence in particular has critical implications for amino acid changes in its RBS needed to quantitatively switch its binding preference to human receptors. In this context, an important unanswered question is how current H5 HA would quantitatively switch to human receptor binding in the context of other molecular changes in its RBS due to sequence divergence from prototypic strains such as Viet04 and Ind05 (Watanabe et al., 2012 Trends In Microbiology, 20:11; Watanabe et al., 2011 PLoS Pathogens, 7:e1002068). This question is of particular importance given the observation that since 2006 H5 HAs isolated have considerably diverged from the prototypic strains Viet04 and Ind05 that have been the focus of many of the studies discussed above.

Described here is a novel framework to systematically investigate RBS of H5 HA by taking into account its sequence evolution and structural topology of its natural avian receptor. Previously we had developed a framework to distinguish binding of HA to avian and human receptor on the basis of the three-dimensional structural topology of these receptors. The avian receptor, when bound to HA, sampled a conformational space that resembles a cone (and hence the term *cone-like* topology was used to describe this receptor). The majority of contacts of H5 HA (using Viet04 crystal structure (Stevens et al., 2006 Science, 312:404; Stevens et al., 2008, J Mol Biol, 381:1382) with avian receptor adopting a cone-like topology involves Neu5Acα2-3Gal- motif. The amino acids in the H5 HA RBS involved in this interaction predominantly lie in the base of the RBS involving residues Ser-136 in 130-loop, Trp-153 and Ile-155 in the 150-loop, Lys-222, Gln-226 in the 220-loop with specific additional contacts from Glu-190, Lys-193 and Leu-194 in the 190-helix a the top of the RBS (**FIG. 2**).

Certain human adapted Has are known, including various seasonal and pandemic strains from H1N1, H3N2 and the pandemic H2N2 subtypes. Based on the phylogenetic 'closeness' of H5 HA to H2 HA (**FIG. 3**), a human-adapted H2N2 HA (A/Albany/6/58 or Alb58) bound to human receptor was selected to contrast with the structural analysis of H5 HA bound to avian receptor (Stevens et al., 2006 Science, 312:404; Stevens et al., 2008, J Mol Biol, 381:1382). The Alb58 strain was selected as a representative H2N2 strain given that it is a prototypic pandemic strain and that its quantitative glycan receptor-binding and phenotypic properties (such as aerosol transmissibility) have been studied. Since the x-ray crystal structure of Alb58 HA is not available, a homology-based model for this HA was constructed using the template crystal structure of another human adapted H2 HA (A/Singapore/1/57) (co-crystallized with human receptor), which has a high sequence identity to Alb58 HA.

*Results*

It was observed that the human receptor bound to HA samples over a larger conformational space that resembles a fully closed to fully open umbrella. Hence the term *umbrella-like* topology may be used to define this receptor. There are two regions in the umbrella-like topology of the human receptor, the base region comprising of Neu5Acα2→6Galβ1→ motif and an extension region comprising sugar residues beyond this motif (typically GlcNAcβ1→3Galβ1→). These two regions span a wider range of interacting amino acids in the H2 HA RBS. A comparison of H5 HA bound to avian receptor in cone-like topology and H2 HA bound to human receptor in umbrella-like topology showed at least four important differences (**FIG. 2**). *First,* the composition of the 130 loop of H2 HA is different from H5 HA because it includes a deletion at position 130 (H3 numbering). *Second,* amino acids in the 'base' of the RBS (such as those in positions 136-138, 219-228) that play a role in interacting with Neu5Acα2→6Galβ1→ motif are different. *Third,* the 'top' of the RBS primarily comprising the '190-helix' (residues 188-196) that interacts with the extension region of human receptor in H2 HA is different (specifically at positions 188, 189, 192 and 193). *Fourth,* position 158 is glycosylated in H5 HA but not H2 HA. Glycosylation at this site could potentially interfere with the extension region of human receptor (*see* Stevens et al., 2008, J Mol Biol, 381:1382).

In this example, these four major differences were categorized into molecular features that distinguish the RBS of H2 and H5 HA. Without wishing to be held to a particular theory, it is possible that making amino acid changes to transform RBS of H5 HA to that of H2 HA by matching these four features would result in a quantitative switch in binding of H5 HA to human receptors.

To understand the effect of designed amino acid changes in the context of other residues in the RBS, a metric (RBS network or RBSN) was developed to capture the network of interactions between the critical residues in the RBS that make contact with the glycan receptor in the appropriate topology and other residues in their close spatial environment. The interactional relationship between critical RBS residues and their environment is represented using a two-dimensional open connectivity network diagram (RBSN diagram). The extent of the interaction network of an amino acid in the RBS is quantified using a normalized network score (RBSN score) that varies from 0 (absence of any network) to 1 (most networked). The higher the network of an amino acid within the RBS, the more it is structurally constrained to be mutated. For the purposes of this example, this network relationship within the RBS guided the process of transforming H5 HA RBS to resemble H2 HA RBS by matching the four features as exemplified in the following.

Matching Feature 1 involved making changes to the 130 loop, specifically by introducing a deletion. The deletion in the 130 loop affects the RBSN diagram involving 131, 133, and 155 positions. Residues at positions 131 and 133 had low RBSN scores (<0.04) in H5 HA and therefore could be readily mutated to match the RBSN diagram involving these positions in H2 HA. Matching Feature 2 involved changes to a combination of residue positions in the 130-loop and 220-loop at the base of the RBS. In H5 HA, Gln-226 plays a critical role in contacts with Neu5Acα2→3Gal- motif of avian receptor and Ser-137 and Gln-226 are involved in inter-residue interaction network.

Conversely, in H2 HA the corresponding Leu-226 and Arg-137 are not related. Arg-137 and Ser-228 in H2 HA provide additional stabilizing contacts with sialic acid. Therefore, according to the present disclosure, one way to match Feature 2 involves changing residues at 137 and 226 positions in H5 HA to match the RBSN diagram of H2 HA. Residue position at 137 was readily mutable given its low RBSN score in H2 and H5 HA (~0.01). However, residue at 226 has a much higher RBSN score in H2 and H5 HA (>0.25). Making changes to this residue therefore also involved making other changes: specifically changing Gly-228→Ser in addition to Ser-137→Arg mutation. While Gln-226→Leu mutation governs switch in contacts from Neu5Acα2→3Gal- to Neu5Acα2→6Gal- motif, Ser-137→Arg and Gly-228→Ser mutation provide additional stabilization to the 130- and 220-loop at the base of the H5 RBS from the standpoint of inter-amino acid networking and improved contacts with glycan receptor ((Stevens et al., 2006 Science, 312:404; Stevens et al., 2008, J Mol Biol, 381:1382). This stabilization may also be accomplished by mutation Asn-224 to Lys or Arg (Arg-224 is observed in pandemic 1918 H1N1 HA) as this would enhance its inter-amino acid interaction network with Asp-96, Leu-97 and Pro-99 (**FIG. 2**). Therefore Feature 2 can also be matched by combining mutations at the 224 position. The RBSN diagram of the residue at 221 position in H5 HA is identical to that in H2 HA, although this position has a Ser in H5 HA and a Pro in H2 HA (**FIG. 2**). It is likely for the Pro to govern the conformation and side chain orientation of the adjacent Lys-222 residue, which plays a key role in making contacts with human receptor in H2 HA. Therefore, changing Ser-221→Pro in H5 HA would permit a more comprehensive matching of Feature 2.

The third feature that distinguished RBS of H2 and H5 HA had many differences in terms of residues at positions 188, 189, 192, and 193 and the RBSN diagrams depicting their interaction networks (**FIG. 2**). The residues Ala-188, Ala-189, and Thr-192 in H5 HA do not have any inter-residue contacts with other residues in the RBS. On the other hand, residues Glu-188, Thr-189 and Arg-192 are involved in multiple interaction networks. Therefore, matching Feature 3 involved making amino changes at positions 188, 189, 192, and 193 in H5 HA to match their corresponding interaction network in H2 HA. Given that the RBSN scores of all these residue positions are low (<0.1) in H5 HA, they are readily mutable. Finally, matching Feature 4 involved removal of glycosylation sequon at position 158. This could be accomplished by mutating Asn-158 to a residue such as Asp or by mutating Thr-160 to Ala. Using these molecular features as a guiding framework, this example demonstrates, for the first time, a quantitative switch in binding of H5 HA to human receptors.

Having described the H5 HA RBS in terms of features that distinguish it from H2 HA RBS, the entire H5 HA sequence space (2,959 full-length non-redundant H5 sequences from GISAID EpiFlu database (platform.gisaid.org/epi3/)) was searched for these features, instead of searching for specific hallmark human-adaptive mutations as described in previous studies (Stevens et al., 2008, J Mol Biol, 381:1382; Russell et al., 2012, Science, 336:1541; Neumann et al., 2012, PLoS pathogens, 8:e1002932; Maines et al., 2011, Virology, 413:139).

The results of this analysis (**FIG. 4**) permitted tracking of these features in the context of the natural evolution of H5 HA and rendered the following four key observations: 1) H5 HAs from many of the recent avian and human isolates (after 2007) have already acquired the deletion in the 130-loop and therefore are closer to matching Feature 1, 2) some of the strains have also acquired changes in the 220 loop such as Asn-224→Lys and Ser221→Pro mutations and therefore are closer to matching Feature 2, 3) key amino acid changes were already observed in the '190-helix' specifically at the 188, 192 and 193 positions making it closer to match Feature 3, and 4) loss of glycosylation at 158 position (Feature 4) is also observed in many H5 HA sequences since 1959. In the context of the key structural features of HA RBS, the deletion in the 130 loop with a concurrent loss of glycosylation (Features 1 and 4) in the same HA was the most critical change observed in the evolution of H5 HA through further diversification of clade 2 sequences after 2007 (**FIG. 4A**). There has been a dramatic increase in percentage of isolates possessing these key features since their initial emergence in 2007. Phylogenetic analysis of the sequences of these isolates showed that they belonged to clade 2.2.1. A relatively smaller percentage of H5N1 isolates have acquired amino acid changes in the '190-helix' closer to match Feature 3 of the H2 RBS (**FIG. 4B**) and these isolates belong to clade 7 (Davis et al., 2010, Avian diseases, 54:307). Notably, we observed that the loss of glycosylation is concomitant with the deletion of 130-loop residue but not vice versa. This finding suggests that specific current H5 HA strains have not only diverged considerably from older human isolates (such as Viet04) but have also acquired key molecular features necessary for matching the pandemic H2 HA RBS.

Based on tracking the molecular features across the natural evolution of H5 HA sequence, HAs from different clades and time periods were chosen to validate our approach. Viet04 HA was the farthest in terms of matching features and therefore was predicted to require the most mutations to match features for the switch. Other HAs were chosen from either clade 2.2.1 or clade 7 which are much closer in terms of matching features with H2 HA RBS and therefore were predicted to require fewer mutations to switch to human receptor-binding. Wild-type and mutant forms of these Has were systematically generated and recombinantly expressed, and their glycan-binding properties were assessed in dose-dependent direct binding assay (25). The results are summarized in **Table 2**.

**Table 2:**

| Human receptor -> | Feature-1 (130-loop deletion) | | | | | | | | | Base (Neu5Acα2-6Galβ1-) Feature-2 (Composition of base) | | | | | | | | | | | | | Extension (-4GlcNAcβ1-3Galβ1-4-) Feature-3 (190-helix) | | | | | | | | | Feature-4 (glycosylation) | | | | α2-3 | α2-6 |
|---|---|---|---|---|---|---|---|---|---|---|---|---|---|---|---|---|---|---|---|---|---|---|---|---|---|---|---|---|---|---|---|---|---|---|---|---|---|---|
| | 127 | 128 | 129 | 130 | 131 | 132 | 133 | 135 | 136 | 137 | 138 | 153 | 155 | 183 | 219 | 221 | 222 | 224 | 225 | 226 | 227 | 228 | 186 | 187 | 188 | 189 | 190 | 192 | 193 | 194 | 196 | 156 | 158 | 159 | 160 | | |
| Alb58 | T | Q | H | - | T | T | T | G | S | R | A | W | T | H | T | P | K | N | G | L | G | S | N | D | E | T | E | R | T | L | Q | K | G | S | N | + | ++++ |
| Viet04 (V2.0) | S | D | H | E | A | S | L | V | S | S | A | W | I | H | T | S | K | N | G | Q | S | G | N | D | A | A | E | T | K | L | Q | K | N | N | T | ++++ | - |
| V2.1 | S | D | H | E | A | S | L | V | S | S | A | W | I | H | T | S | K | N | G | L | S | S | N | D | A | A | E | T | R | L | Q | K | N | N | A | +++ | +++ |
| V2.2 | S | D | H | E | A | S | L | V | S | S | A | W | I | H | T | S | K | K | G | L | S | G | N | D | A | A | E | T | K | L | Q | K | N | N | A | - | + |
| V2.3 | S | D | H | - | T | T | T | G | S | R | A | W | T | H | T | P | K | N | G | L | G | S | N | D | E | A | E | R | A | L | Q | K | N | N | T | - | ++++ |
| Egy06 (E3.0) | S | D | H | E | A | S | S | V | S | S | A | W | I | H | T | S | K | N | G | Q | S | G | N | D | A | A | E | T | R | L | Q | K | D | N | A | ++++ | - |
| E3.1 | S | D | H | E | A | S | S | V | S | S | A | W | I | H | T | S | K | N | G | L | S | S | N | D | A | A | E | T | R | L | Q | K | D | N | A | +++ | +++ |
| Egy09 (E4.0) | S | D | H | - | E | A | S | V | S | S | A | W | T | H | T | S | K | N | G | Q | S | G | N | D | A | T | E | T | R | L | Q | K | N | N | A | ++++ | - |
| E4.1 | S | D | H | - | E | A | S | V | S | R | A | W | T | H | T | S | K | N | G | L | S | S | N | D | A | T | E | T | R | L | Q | K | N | N | A | + | ++ |
| E4.2 | S | D | H | - | E | T | S | V | S | R | A | W | T | H | T | P | K | N | G | L | S | S | N | D | A | T | E | T | T | L | Q | K | N | N | A | + | +++ |
| E4.3 | S | D | H | - | E | A | S | V | S | S | A | W | T | H | T | S | K | K | G | L | S | G | N | D | A | T | E | T | R | L | Q | K | N | N | A | - | +++ |
| dkEgy10(E5.0) | S | D | H | - | E | A | S | V | S | S | A | W | T | H | T | S | K | K | G | G | S | G | N | D | A | T | E | T | R | L | Q | K | D | N | A | +++ | - |
| E5.1 | S | D | H | - | E | A | S | V | S | S | A | W | T | H | T | S | K | K | G | L | S | G | N | D | A | T | E | T | R | L | Q | K | D | N | A | - | ++ |
| ckViet08 (V4.0) | S | N | H | E | T | S | L | V | S | S | A | W | T | H | T | S | K | N | G | Q | S | G | N | N | E | A | E | K | M | I | Q | K | N | N | T | +++ | - |
| V4.1 | S | N | H | E | T | S | L | V | S | S | A | W | T | H | T | S | K | N | G | L | S | S | N | N | E | A | E | K | M | I | Q | K | N | N | T | - | - |
| V4.2 | S | N | H | E | T | S | L | V | S | S | A | W | T | H | T | S | K | N | G | L | S | S | N | N | E | A | E | K | M | I | Q | K | N | N | A | ++ | ++ |
| V4.3 | S | N | H | - | T | S | T | V | S | R | A | W | T | H | T | S | K | N | G | L | S | S | N | D | E | A | E | K | T | I | Q | K | N | N | A | + | ++++ |

The residue positions corresponding to Features 1, 2, 3 and 4 are shown with the amino acids. The amino acid mutations are highlighted in *gray*. The residues involved in the inter-residue interaction networks depicted by the RBSN diagram in **FIG. 2** are shown in the same color. For example, residue positions 131, 133 and 155 are colored the same given that 131 and 155 are involved in the interaction network in H2 RBS and 133 and 155 are involved in the interaction network in H5 RBS. Residue positions outside the RBS are not shown (although they may be involved in the interaction networks of RBS residues). The affinities are indicated using '+' symbol, where highest = '++++', high = '+++', moderate = '++' and low = '+'. Absence of any observable binding is indicated using '-'. Although human receptor-binding of V2.2 was relatively much higher than its corresponding avian receptor binding, the signals were too low to interpret this behavior as a quantitative switch. The relative binding affinities of the H5 HA mutants that quantitatively switch binding preference to human receptors are shown in *green* box in the same way as the reference wild-type Alb58 H2 HA

As predicted by the model described in this example, 13 amino acid changes made to comprehensively match Features 1, 2 and 3 (including incorporation of inter-residue interaction network) when made on Viet04 (V2.3 in **Table 2**) quantitatively switched its binding to human receptor when compared to wild-type (**FIG. 5**). The binding affinity of V2.3 to human receptor quantified using an apparent binding constant Kd' as described previously (Srinivasan et al., 2008, Proc Natl Acad Sci, 105:2800) (Kd' ~ 3 pM) was in the same range as that calculated for 1918 H1N1 HA (Srinivasan et al., 2008, Proc Natl Acad Sci, 105:2800). While partially matching one or two features such as Feature 2 (Gln-226→Leu/Gly-228→Ser) and 4 (Thr-160→Ala) (V2.1 in **Table 2**) showed substantially increased binding to human receptors, this mutant also retained high affinity binding to avian receptor, which is not characteristic of a quantitative switch. Matching Feature 2 was also investigated through the alternate strategy of improving inter-residue and RBS-glycan contacts involving the 220-loop by introducing Gln-226→Leu/Asn-224→Lys mutations in the context of matching Feature 4 (Thr-160→Ala (V2.2 in **Table 2**). This mutant showed very low glycan-receptor binding and hence it was not possible to determine binding affinity for qualification as quantitative switch. Therefore, in this example, and in the case of clade 1 HA such as Viet04, matching at three Features at minimum may be required for a quantitative switch.

Among clade 2.2.1 HAs, a recent human isolate A/Egypt/N03450/2009 (or Egy09) was selected for further study because it was found to be the best representative strain in 2.2.1, due to its high sequence similarity to clade 2.2.1 consensus sequence. Egy09 HA already had deletion at 130-loop, loss of glycosylation sequon at the 158 position thereby matched Features 1 and 4 (**Table 2**). Furthermore, the RBSN diagram involving Glu-131, Ser-133, and Thr-155 in Egy09 HA was identical to the corresponding diagram involving Thr-131, Thr-133, and Thr-155 in Alb58 HA (RBSN diagram not shown). In parallel, RBSN diagram involving residues Ala-188 and Thr-192 in Egy09 was similar to the corresponding diagram involving residues Glu-188 and Arg-192 in H2 HA RBS. Consequently, as predicted by the "feature-matching" analyses of this example, fewer amino acid changes were required in Egy09 HA to match all the four Features (E4.2 in **Table 2**) to quantitatively switch its binding to human receptors (**FIG. 6A and 6B**). The human receptor affinity of E4.2 was quantified by Kd'~25 pM. Matching Feature 2 through the alternate strategy of introducing Gln-226→Leu and Asn224→Lys in Egy09 resulted in a mutant HA where Features 1, 2, and 4 are matched (E4.3) that also showed an observable quantitative switch from avian to human receptor binding (FIG. 6C). The binding affinity of E4.3 for human receptor was ~50 pM. Therefore, even in the case of clade 2.2.1, matching 3 out of the 4 features quantitatively switched its binding preference to human receptor. Moreover, the RBS of Egy09 appears to have evolved such that only two amino acid changes were sufficient to match 3 out of 4 Features to achieve the switch with characteristic human receptor binding that is necessary for viral transmission.

In light of our success in matching Feature 2 with just 2 amino acid changes in Egy09 and the fact that Asn-224→Lys has been observed in some of the recent H5N1 isolates, we sought to determine if this mutation naturally occurred in any of the clade 2.2.1 HA sequences. The search resulted in select sequences among which we chose another clade 2.2.1 HA - A/duck/Egypt/10185SS/2010 (Egy10) HA which would require just the Gln→226 Leu change to match 3 out of the 4 features. Consistent with this prediction Egy10 required just a single base-pair mutation leading to Gln-226→Leu change to quantitatively switch its binding preference to human receptor with binding affinity Kd' ~ 100 pM (**FIG. 6D**) in a fashion similar to the 2009 H1N1 pandemic HA and passes the necessary threshold for viral transmission (**FIG. 1**).

Among the clade 7 HAs, chicken/Vietnam/NCVD-093/08 (ckViet08) was selected because it appeared to be the best representative of this clade and has already acquired amino acid changes in the '190-helix' specifically at positions 188, 192 and 193 so as to bring it closer to match Feature 3 of H2 HA RBS. On this HA, systematic amino acid changes to match Feature 1 (introduction of 130-loop deletion and Leu-133→Thr mutation), Feature 2 (*LS* + Ser137→Arg), Feature 3 (Asn-187→Asp and Met-193→Thr), and Feature 4 (loss of glycosylation at 158) quantitatively switched its binding preference to human receptors (V4.3 in **Table 2**).

In summary, using a novel approach to define the molecular features that characterize RBS of H5 HA, this example demonstrates that HAs from specific recent clade 2.2.1 H5N1 isolates require only a single Gln226→Leu amino acid mutation to quantitatively switch its binding to human receptor. The approach described in this example emphasizes the need to analyze RBS features that would quantitatively switch binding to human receptor. It is evident from this approach that for a given H5 HA there are distinct ways to match RBS features and that the number of amino acids required for human adaptation is a variable parameter that critically depends on the natural sequence evolution of H5 HA. In fact, when the same amino acid changes that conferred aerosol transmissibility to Ind05 virus in ferrets were introduced in a representative clade 2.2.1 HA, dramatically different glycan binding properties were observed (**FIG. 7**). Furthermore, matching Feature 2 (Asn-224→Lys and Gly226→Leu mutations) and Feature 4 alone in the absence of the 130-loop deletion in clade 1 HA such as Viet04 showed substantially low glycan binding signals. This finding underscores the significance of the inventive approach, capturing the RBS features in the context of H5 HA evolution, rather than simply using specific amino acid changes reported for Viet04 (Imai et al., 2012, Nature, 486:420) or Ind05 (Herfst et al., 2012, Science, 336:1534) as starting points for human adaptation of all H5 HAs.

Phylogenetic analysis of the H5N1 HAs that have naturally acquired Features 1 & 4 showed that they belong to clade 2.2.1 and those that have acquired Feature 3 belong to clade 7. It is worth noting that HA from the strains belonging to these clades are closer to human adaptation. Importantly, apart from Ser137→Arg, Gln226→Leu and Gly228→Ser, all the other amino acid changes and also the deletion in the 130-loop have been observed in H5N1 (in the more recent strains after 2006) and these changes have also sustained the evolution of H5N1 HA sequences. Therefore, one prospective surveillance strategy revealed as desirable by this example could involve monitoring the co-occurrence of these changes in the currently circulating H5 HAs. It should be noted that a majority of current influenza surveillance efforts are focused on the Asian subcontinent (China, Vietnam, and Thailand). However, all of the human H5N1 isolates belonging to clade 2.2.1 are from Egypt and Israel. It will be important to closely monitor the evolution of the clade 2.2.1 and clade 7 strains. Much of the focus on human H5N1 adaptation and vaccine strategies has been around strains from 2003-2006. The five currently approved human H5N1 vaccines for clinical use are also based on strains from earlier clades.

In this example, a metric was used that compares antigenic identity between HAs (Tharakaraman, K. et al.; manuscript submitted) in order to correlate antigenic identity with cross reactive neutralizing anti-sera response in ferrets. This analysis showed that showed that low antigenic identity correlated with poor cross protection. Extending this analysis we showed that strains from 2003-2006 such as Viet04 and Ind05 share low antigenic identity with clade 2.2.1 and clade 7. Currently approved human H5N1 vaccines are therefore unlikely to effectively protect infection by wild-type and mutant forms of clade 2.2.1 and clade 7 strains described in this study. The results from our approach offer insights that could potentially be valuable for the surveillance of evolution of current H5N1 strains and could also augment existing vaccine strategies for better pre-pandemic preparedness in the event of a possible outbreak of H5N1 in the human population.

*Materials and Methods*

*Cloning, baculovirus synthesis, expression and purification of HA*

H5 WT and variant HA sequences were codon-optimized for insect cell expression and synthesized at DNA2.0 (Menlo Park, CA). The synthesized genes were then sub-cloned into pAcGP67A plasmid and baculoviruses were created using Baculogold system (BD Biosciences, San Jose, CA) according to manufacturer's instructions. The recombinant baculoviruses were then used to infect suspension cultures of Sf9 cells cultured in BD Baculogold Max-XP SFM (BD Biosciences, San Jose, CA). The infection was monitored and the conditioned media was harvested 3-4 days post-infection. The soluble HA from the harvested conditioned media was purified using Nickel affinity chromatography (His-Trap HP columns, GE Healthcare, Piscataway, NJ). Eluting fractions containing HA were pooled, concentrated and buffer exchanged into IX PBS pH 8.0 (Gibco) using 100K MWCO spin columns (Millipore, Billerica, MA). The purified protein was quantified using BCA method (Pierce).

The gene was codon optimized for mammalian expression, synthesized (DNA2.0, Menlo Park, CA) and sub-cloned into modified pcDNA3.3 vector for expression under CMV promoter. Recombinant expression of HA was carried out in HEK 293-F FreeStyle suspension cells (Invitrogen, Carlsbad, CA) cultured in 293-F FreeStyle Expression Medium (Invitrogen, Carlsbad, CA) maintained at 37 °C, 80% humidity and 8% $CO_2$. Cells were transfected with Poly-ethylene-imine Max (PEI-MAX, PolySciences, Warrington, PA) with the HA plasmid and were harvested seven days post-infection. The supernatant was collected by centrifugation, filtered through a 0.45 $\mu$m filter system (Nalgene, Rochester, NY) and supplemented with 1:1000 diluted protease inhibitor cocktail (Calbiochem filtration and supplemented with 1:1000 diluted protease inhibitor cocktail (EMD Millipore, Billerica, MA). HA was purified from the supernatant using His-trap columns (GE Healthcare) on an AKTA Purifier FPLC system. Eluting fractions containing HA were pooled, concentrated and buffer exchanged into IX PBS pH 7.4 using 100K MWCO spin columns (Millipore, Billerica, MA). The purified protein was quantified using BCA method (Pierce, Rockford, IL).

Both expression systems were used in this example. Importantly, no differences were observed in the glycan binding properties of the HA derived from baculovirus when compared to that of the material derived from mammalian expression.

*Homology Modeling of HA*

A structural model of Alb58 HA trimer was built using the MODELLER homology modeling software. To build the model, the solved crystal structure of A/Singapore/1/57 hemagglutinin with human receptor (PDB: 2WR7), which has 99% sequence identity in HA1 to Alb58, was used as a template. During modeling, the ligand (human receptor) was copied from the template structure into the model structure. The final model was minimized to release internal constraints.

*Dose dependent direct binding of WT and variant HA to HA Receptor Glycans*

To investigate the multivalent HA-glycan interactions a streptavidin plate array comprising representative biotinylated α2→3 and α2→6 sialylated glycans was used as described previously. 3'SLN, 3'SLN-LN, 3'SLN-LN-LN are representative avian receptors. 6'SLN and 6'SLN-LN are representative human receptors. LN corresponds to lactosamine (Galβ1-4GlcNAc) and 3'SLN and 6'SLN respectively correspond to Neu5Acα2-3 and Neu5Acα2-6 linked to LN. The biotinylated glycans were obtained from the Consortium of Functional Glycomics through their resource request program. Streptavidin-coated High Binding Capacity 384-well plates (Pierce) were loaded to the full capacity of each well by incubating the well with 50 μl of 2.4 μM of biotinylated glycans overnight at 4 °C. Excess glycans were removed through extensive washing with PBS. The trimeric HA unit comprises of three HA monomers (and hence three RBS, one for each monomer). The spatial arrangement of the biotinylated glycans in the wells of the streptavidin plate array favors binding to only one of the three HA monomers in the trimeric HA unit. Therefore in order to specifically enhance the multivalency in the HA-glycan interactions, the recombinant HA proteins were pre-complexed with the primary and secondary antibodies in the molar ratio of 4:2:1 (HA:primary:secondary). The identical arrangement of 4 trimeric HA units in the precomplex for all the HAs permits comparison between their glycan binding affinities. A stock solution containing appropriate amounts of Histidine tagged HA protein, primary antibody (Mouse anti 6X His tag IgG from Abcam) and secondary antibody (HRP conjugated goat anti Mouse IgG from Santacruz Biotechnology) in the ratio 4:2:1 and incubated on ice for 20 min. Appropriate amounts of precomplexed stock HA were diluted to 250 μl with 1% BSA in PBS. 50 μl of this precomplexed HA was added to each of the glycan-coated wells and incubated at room temperature for 3 hrs followed by the wash steps with PBS and PBST (1X PBS + 0.05% Tween-20). The binding signal was determined based on HRP activity using Amplex Red Peroxidase Assay kit (Invitrogen, CA) according to the manufacturer's instructions. The experiments were done in triplicate. Minimal binding signals were observed in the negative controls including binding of pre-complexed unit to wells without glycans and binding of the antibodies alone to the wells with glycans. The binding parameters, cooperativity (n) and apparent binding constant (Kd'), for HA-glycan binding were calculated by fitting the average binding signal value (from the triplicate analysis) and the HA concentration to the linearized form of the Hill equation:

$$\log\left(\frac{y}{1-y}\right) = n * \log([HA]) - \log\left(K_d{'}\right)$$

where y is the fractional saturation (average binding signal/maximum observed binding signal). In order to compare Kd' values, the values reported in this study correspond to the appropriate representative avian (3'SLN-LN or 3'SLN-LN-LN) and human (6'SLN-LN) receptor that gave the best fit to the above equation and the same slope value (n ~1.3).

As noted above, there were no differences in the glycan binding properties for HA derived from baculovirus when compared to that of HA produced via mammalian expression.

*Capturing Network of RBS residues (RBSN).*

The coordinates of H5 HA - avian receptor and Alb58 HA - human receptor complex was uploaded into the PDBePISA server (http://www.ebi.ac.uk/msd-srv/prot_int/pistart.html) to determine key residues in the HA RBS that make contact with the corresponding glycan receptor (interface cut-off of 30% was used). For these residues, their environment was defined using a distance threshold of 7 Å and the contacts including putative hydrogen bonds (including water-bridged ones), disulfide bonds, pi-bonds, polar interactions, salt bridges, and Van der Waals interactions (non-hydrogen) occurring between pairs of residues within this threshold distance was computed as described previously (Soundararajan et al., 2011, Sci. Rep., 1). These data were assembled into an array of eight atomic interaction matrices. A weighted sum of the eight atomic interaction matrices were then computed to produce a single matrix that accounts for the strength of atomic interaction between residue pairs within the RBS, using weights derived from relative atomic interaction energies (Soundararajan et al., 2011, Sci. Rep., 1). The inter-residue interaction network calculated in this fashion generates a matrix that describes all the contacts made by critical RBS residues with spatial proximal neighboring residues in their environment. Each element *i, j* is the sum of the path scores of all paths between residues *i* and *j*. The degree of networking score for each residue was computed by summing across the rows of the matrix, which was meant to correspond to the extent of "networking" for each residue. The degree of networking score was normalized (RBSN score) with the maximum score for each protein so that the scores varied from 0 (absence of any network) to 1 (most networked).

*Sequence analysis of H5 HA and estimation of key features*

A total of 6,014 H5 HA sequences were downloaded from the EpiFlu database. From this, only those sequences that had complete coding regions including start and stop codons were considered. In order to avoid estimation errors due to multiply represented sequences, all groups of identical sequences in the dataset were represented by the oldest sequence in the group. The remaining 2,959 sequences were ordered by isolation time, aligned, and the occurrence rate of each feature (defined as the percent fraction of sequences from a given year that contains that feature) was calculated.

**Claims**

1.  An engineered H5 HA polypeptide of wild type A/Egypt/N03450/2009 (Egy09); wherein, in the sequence element NGQSG, "N" is changed to "K" and "Q" is changed to "L".

2.  An engineered H5 HA polypeptide of wild type A/duck/Egypt/10185SS/2010 (Egy10); wherein, in the sequence element KGQSG, "Q" is changed to "L".

3.  A vaccine composition comprising at least one antigen that is a polypeptide of claim 1 or claim 2 and a pharmaceutically acceptable carrier.

4.  A method of providing a vaccine comprising providing at least one antigen comprising a polypeptide of claim 1 or claim 2; and formulating the provided at least one antigen into a vaccine composition.

5.  A diagnostic kit for determining pandemic risk in a strain of H5 influenza, the kit comprising at least one antibody that specifically binds to a polypeptide of claim 1 or claim 2.

6.  A method of monitoring influenza in a sample from a source:

    a. contacting the sample suspected to contain influenza with one or more agents that specifically binds to an H5 HA polypeptide of claim 1 or claim 2; and
    b. detecting binding of the agent with the sample, so that presence and/or level of the H5 HA in the sample is determined.

7.  The method of claim 6, wherein the source is an environmental source.

8.  The method of claim 6, wherein the source is a human patient.

9.  The method of claim 6, wherein the obtaining, contacting and detecting steps are repeated at least once after a period of time has elapsed since the first obtaining, contacting and detecting steps were completed.

10. The method of claim 6, further comprising contacting a sample from the source with one or more agents that specifically bind to an H5 HA that does not infect humans.

**Patentansprüche**

1.  Gentechnisch verändertes H5-HA-Polypeptid von Wildtyp A/Egypt/N03450/2009 (Egy09), wobei in dem Sequenzelement NGQSG "N" zu "K" verändert ist und "Q" zu "L" verändert ist.

2.  Gentechnisch verändertes H5-HA-Polypeptid von Wildtyp A/duck/Egypt/10185SS/2010 (Egy10), wobei in dem Sequenzelement KGQSG "Q" zu "L" verändert ist.

3.  Impfstoffzusammensetzung, umfassend mindestens ein Antigen, das ein Polypeptid nach Anspruch 1 oder Anspruch 2 ist, und einen pharmazeutisch akzeptablen Träger.

4.  Verfahren zum Bereitstellen eines Impfstoffs, umfassend das Bereitstellen mindestens eines Antigens, das ein Polypeptid nach Anspruch 1 oder Anspruch 2 umfasst, und Formulieren des bereitgestellten mindestens einen Antigens zu einer Impfstoffzusammensetzung.

5.  Diagnostik-Kit zur Bestimmung des Pandemierisikos bei einem H5-Influenza-Stamm, wobei das Kit mindestens einen Antikörper umfasst, der spezifisch an ein Polypeptid nach Anspruch 1 oder Anspruch 2 bindet.

6.  Verfahren zur Kontrolle von Influenza in einer Probe aus einer Quelle:

a. Inkontaktbringen der Probe, von der vermutet wird, dass sie Influenza enthält, mit einem oder mehreren Mitteln, das/die spezifisch an ein H5-HA-Polypeptid nach Anspruch 1 oder Anspruch 2 bindet/binden; und
b. Nachweisen der Bindung des Mittels an die Probe, so dass das Vorhandensein und/oder der Spiegel von H5-HA in der Probe bestimmt wird.

**7.** Verfahren nach Anspruch 6, wobei die Quelle eine Umweltquelle ist.

**8.** Verfahren nach Anspruch 6, wobei die Quelle ein menschlicher Patient ist.

**9.** Verfahren nach Anspruch 6, wobei die Schritte des Erhaltens, Inkontaktbringens und Nachweisens mindestens einmal wiederholt werden, nachdem ein Zeitraum nach dem Abschluss der ersten Schritte des Erhaltens, Inkontaktbringens und Nachweisens vergangen ist.

**10.** Verfahren nach Anspruch 6, das ferner das Inkontaktbringen einer Probe aus der Quelle mit einem oder mehreren Mitteln umfasst, das/die spezifisch an ein H5-HA, das Menschen nicht infiziert, bindet/binden.

## Revendications

**1.** Polypeptide génétiquement manipulé HA de H5 du type sauvage A/Egypt/N03450/2009 (Egy09) ;
dans lequel, dans l'élément de séquence NGQSG, "N" est changé en "K" et "Q" est changé en "L".

**2.** Polypeptide génétiquement manipulé HA de H5 du type sauvage A/duck/Egypt/10185SS/2010 (Egy10) ;
dans lequel, dans l'élément de séquence KGQSG, "Q" est changé en "L".

**3.** Composition vaccinale comprenant au moins un antigène, qui est un polypeptide de la revendication 1 ou la revendication 2, et un véhicule acceptable d'un point de vue pharmaceutique.

**4.** Procédé de fourniture d'un vaccin comprenant les étapes consistant à fournir au moins un antigène comprenant un polypeptide de la revendication 1 ou la revendication 2 ; et formuler l'au moins un antigène fourni dans une composition vaccinale.

**5.** Trousse diagnostique destinée à déterminer un risque pandémique dans une souche de grippe H5, la trousse comprenant au moins un anticorps qui se lie spécifiquement à un polypeptide de la revendication 1 ou la revendication 2.

**6.** Procédé de suivi d'une grippe dans un échantillon à partir d'une source, consistant à :

a. mettre en contact l'échantillon suspecté de contenir une grippe avec un ou plusieurs agent(s) qui se lie/lient spécifiquement à un polypeptide HA de H5 de la revendication 1 ou la revendication 2 ; et
b. détecter une liaison de l'agent avec l'échantillon, de sorte que la présence et/ou le taux du HA de H5 soit déterminé (e) dans l'échantillon.

**7.** Procédé de la revendication 6, dans lequel la source est une source environnementale.

**8.** Procédé de la revendication 6, dans lequel la source est un patient humain.

**9.** Procédé de la revendication 6, dans lequel les étapes d'obtention, de mise en contact et de détection sont répétées au moins une fois après qu'une période de temps se soit écoulée depuis que les premières étapes d'obtention, de mise en contact et de détection ont été terminées.

**10.** Procédé de la revendication 6, comprenant en outre une mise en contact d'un échantillon à partir de la source avec un ou plusieurs agent(s) qui se lie/lient spécifiquement à un HA de H5 qui n'infecte pas les êtres humains.

FIG. 1A

FIG. 1B

FIG. 1C

FIG. 1D

[HA] µg/ml

RELATIVE BINDING SIGNAL (%)

3'SLN
6'SLN
3'SLN-LN
6'SLN-LN
3'SLN-LN-LN

FIG. 2

EP 2 953 967 B1

FIG. 3

FIG. 4A

FIG. 4B

FIG. 5A

FIG. 5B

FIG. 6A

FIG. 6B

FIG. 6C

FIG. 6D

FIG. 7A

FIG. 7B

FIG. 8B

FIG. 8A

FIG. 9

FIG. 10

α2-6 ATTACHED TO CORE GalNAc
IN CORE 1 TYPE STRUCTURE

α2-6 ATTACHED TO BRANCHED CORE 2
AND CORE 4 STRUCTURES

GLYCOLIPIDS

GLUCOSYLCERAMIDE CORE
ISOGLOBO TYPE

GLUCOSYLCERAMIDE CORE
LACTO TYPE

GLUCOSYLCERAMIDE CORE
GANGLIO TYPE

GLUCOSYLCERAMIDE CORE
GLOBO TYPE

GLUCOSYLCERAMIDE CORE
NEOLACTO TYPE

GLUCOSYLCERAMIDE CORE
GLOBO TYPE

KEY: ■ GlcNAc ☐ GalNAc ○ Gal ● Glc ◆ Neu5Ac ▼ Fucose ▨ Terminal HexNAc

FIG. 10
CONTINUED

CORE 1 O-LINKED GLYCANS

CORE 2 O-LINKED GLYCANS

CORE 3 O-LINKED GLYCANS

CORE 4 O-LINKED GLYCANS

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 087332 A
- US 0930056 W
- US 0718160 W
- WO 9111465 A, Goldenberg
- US 4166452 A
- US 4816567 A
- US 4886499 A
- US 5190521 A
- US 5328483 A
- US 5527288 A
- US 4270537 A
- US 5015235 A
- US 5141496 A
- US 5417662 A
- WO 9934850 A
- US 5480381 A
- US 5599302 A
- US 5334144 A

- US 5993412 A
- US 5649912 A
- US 5569189 A
- US 5704911 A
- US 5383851 A
- US 5893397 A
- US 5466220 A
- US 5339163 A
- US 5312335 A
- US 5503627 A
- US 5064413 A
- US 5520639 A
- US 4596556 A
- US 4790824 A
- US 4941880 A
- US 4940460 A
- WO 9737705 A
- WO 9713537 A

**Non-patent literature cited in the description**

- **ALTSCHUL et al.** Basic local alignment search tool. *J. Mol. Biol.,* 1990, vol. 215 (3), 403-410
- **ALTSCHUL et al.** *Methods in Enzymology*
- **ALTSCHUL et al.** Gapped BLAST and PSI-BLAST: a new generation of protein database search programs. *Nucleic Acids Res.,* 1997, vol. 25, 3389-3402
- **BAXEVANIS et al.** Bioinformatics : A Practical Guide to the Analysis of Genes and Proteins. Wiley, 1998
- Bioinformatics Methods and Protocols. Methods in Molecular Biology. Humana Press, 1999, vol. 132
- **UIPRASERTKUL et al.** *Emerg. Infect. Dis.,* 2005, vol. 11, 1036
- **RUSSELL et al.** *Virology,* 2004, vol. 325, 287
- **SKEHEL.** Annu Rev Biochem. Wiley, 2000, vol. 69, 531
- **ROGERS ; PAULSON.** *Virology,* 1983, vol. 127, 361
- **ROGERS et al.** *Nature,* 1983, vol. 304, 76
- **SAUTER et al.** *Biochemistry,* 1992, vol. 31, 9609
- **CONNOR et al.** *Virology,* 1994, vol. 205, 17
- **TUMPEY et al.** *Science,* 2005, vol. 310, 77
- **EISEN et al.** *Virology,* 1997, vol. 232, 19

- **HA et al.** *Proc Natl Acad Sci USA,* 2001, vol. 98, 11181
- **HA et al.** *Virology,* 2003, vol. 309, 209
- **GAMBLIN et al.** *Science,* 2004, vol. 303, 1838
- **STEVENS et al.** *Science,* 2004, vol. 303, 1866
- **RUSSELL et al.** *Glycoconj J,* 2006, vol. 23, 85
- **STEVENS et al.** *Science,* 2006, vol. 312, 404
- **XU R et al.** *J Virol,* 2010, vol. 84 (4), 1715
- **LIU J et al.** *Proc Natl Acad Sci U S A,* 2009, vol. 106 (40), 17175
- **HARLOW ; LANE.** Antibodies: A Laboratory Manual. 1988
- **SCHADE et al.** *ALTEX,* 1996, vol. 13 (5), 80-85
- **LOSMAN et al.** *Int. J. Cancer,* 1990, vol. 46, 310
- **MILSTEIN ; CUELLO.** *Nature,* 1983, vol. 305 (5934), 537-40
- **RIECHMANN et al.** *Nature,* 1988, vol. 332 (6162), 323-7
- **VERHOEYEN et al.** *Science,* 1988, vol. 239 (4847), 1534-6
- **HOOGENBOOM et al.** *Mol Immunol.,* 1991, vol. 28 (9), 1027-37

- **MARKS et al.** *J Mol Biol.,* 1991, vol. 222 (3), 581-97
- **REISFELD ; SELL.** *Cancer Surv.,* 1985, vol. 4 (1), 271-90
- **FISHWILD et al.** High-avidity human IgG kappa monoclonal antibodies from a novel strain of minilocus transgenic mice. *Nat Biotechnol.,* July 1996, vol. 14 (7), 845-51
- **LONBERG et al.** Antigen-specific human antibodies from mice comprising four distinct genetic modifications. *Nature,* 28 April 1994, vol. 368 (6474), 856-9
- **LONBERG ; HUSZAR.** Human antibodies from transgenic mice. *Int. Rev. Immunol.,* 1995, vol. 13 (1), 65-93
- **MARKS et al.** By-passing immunization: building high affinity human antibodies by chain shuffling. *Biotechnology,* July 1992, vol. 10 (7), 779-83
- **TUERK et al.** *Science,* 1990, vol. 249, 505
- **JAYASENA.** *Clin. Chem.,* 1999, vol. 45, 1628
- **HARLOW ; LANE.** Antibodies: A Laboratory Manual. CSHL Press, 1988
- **A. R. GENNARO.** Remington's The Science and Practice of Pharmacy. Lippincott, Williams & Wilkins, 2006
- **ALLISON.** *Dev. Biol. Stand.,* 1998, vol. 92, 3-11
- **UNKELESS et al.** *Annu. Rev. Immunol.,* 1998, vol. 6, 251-281
- **PHILLIPS et al.** *Vaccine,* 1992, vol. 10, 151-158
- Remington's Pharmaceutical Sciences. Mack Publishing Co, 1995
- Textbook of Influenza. Blackwell Science, 1998
- **SCHILD et al.** *Bull. World Health Organ.,* 1975, vol. 52, 43-50, 223-31
- **MOSTOW et al.** *J. Clin. Microbiol.,* 1975, vol. 2, 531
- **BAYLOR et al.** *Vaccine,* 2002, vol. 20, 18
- **RIBI et al.** Immunology and Immunopharmacology of bacterial endotoxins. Plenum Publ. Corp, 1986, 407
- **COOPER et al.** *Vaccine,* 2004, vol. 22, 3136
- **GHOCHIKYAN et al.** *Vaccine,* 2006, vol. 24, 2275
- **PAYNE et al.** *Vaccine,* 1998, vol. 16, 92
- **CAO et al.** *Vaccine,* 1992, vol. 10, 238
- **KATZ et al.** *Vaccine,* 2000, vol. 18, 2177
- **MBWUIKE et al.** *Vaccine,* 1990, vol. 8, 347
- **KREUTER et al.** *J. Pharm. Sci.,* 1981, vol. 70, 367
- **TUMPEY et al.** *Science,* 2007, vol. 315, 655-659
- **VAN HOEVEN et al.** *PNAS,* 2009, vol. 106, 3366
- **NEUMANN et al.** *Cell Res.,* 2010, vol. 20, 51
- **GUAN et al.** *Rev Sci Tech,* 2009, vol. 28, 39
- **GE et al.** *Crit Rev Microbiol.,* 2011, vol. 37, 157
- **TUMPEY et al.** *Science,* 2007, vol. 315, 655
- **SRINIVASAN et al.** *PNAS,* 2008, vol. 105, 2800
- **PAPPAS et al.** *PLoS One,* 2010, vol. 5, e11158
- **VISWANATHAN et al.** *PLoS One,* 2010, vol. 5, e13768
- **SHRIVER et al.** *Chem Biol.,* 2009, vol. 16, 803
- **GAMBARYAN et al.** *Virology,* 2006, vol. 344, 432
- **YAMADA et al.** *Nature,* 2006, vol. 444, 378
- **CHANDRASEKARAN et al.** *Nat Biotechnol.,* 2008, vol. 26, 107
- **STEVENS et al.** *J Mol. Biol.,* 2008, vol. 381, 1382
- **WANG et al.** *PNAS,* 2009, vol. 106, 18137
- **WATANABE et al.** *Trends In Microbiology,* 2012, vol. 20, 11
- **IMAI et al.** *Nature,* 2012
- **HERFST et al.** *Science,* 2012, vol. 336, 1534
- **RUSSELL et al.** *Science,* 2012, vol. 336, 1541
- **WATANABE et al.** *PLoS Pathogens,* 2011, vol. 7, e1002068
- **STEVENS et al.** *J Mol Biol,* 2008, vol. 381, 1382
- **NEUMANN et al.** *PLoS pathogens,* 2012, vol. 8, e1002932
- **MAINES et al.** *Virology,* 2011, vol. 413, 139
- **DAVIS et al.** *Avian diseases,* 2010, vol. 54, 307
- **SRINIVASAN et al.** *Proc Natl Acad Sci,* 2008, vol. 105, 2800
- **IMAI et al.** *Nature,* 2012, vol. 486, 420
- **SOUNDARARAJAN et al.** *Sci. Rep.,* 2011, 1